# EUROPEAN PATENT APPLICATION

(11) **EP 4 597 308 A1**
(43) Date of publication of application: **06.08.2025**
(21) Application number: 25153741.1
(22) Date of filing: 24.01.2025
(51) Int. Cl.: G06F 8/75, G01N 35/00, G06F 9/445, G06F 11/3668, G06F 11/3698, G16H 10/40, G16H 40/40, G16H 40/63, G16H 40/67

(54) **METHOD FOR CONFIGURING TEST PROJECT AND SAMPLE ANALYSIS SYSTEM**

(30) Priority: 31.01.2024 CN 202410157141
(71) Applicant: Shenzhen New Industries Biomedical Engineering Co., Ltd., Shenzhen, Guangdong 518122 (CN)
(72) Inventor: XIAO, Hao, Guangdong, 518122 (CN); QIN, Heyang, Guangdong, 518122 (CN); YIN, Li, Guangdong, 518122 (CN); TANG, Junhui, Guangdong, 518122 (CN); ZHU, Liang, Guangdong, 518122 (CN); WANG, Yixian, Guangdong, 518122 (CN); LI, Zhirui, Guangdong, 518122 (CN)
(74) Representative: Zacco GmbH

(57) **Abstract**

The disclosure relates to a method and apparatus for configuring a test project, a non-transitory computer-readable storage medium, a control terminal and a sample analysis system. The method includes: acquiring a program mapping relation of a target test project, where the program mapping relation includes mapping relations between a plurality of target sample types and at least one target first experimental program; acquiring program configuration information of a target second experimental program, where the target second experimental program uniquely corresponds to a project type of the target test project; and configuring the target test project on the basis of the program mapping relation and the program configuration information of the target second experimental program. The solution of the disclosure can improve convenience and efficiency of project configuration, and efficiency and accuracy of sample registration.

## Description

### Cross-Reference to Related Application

This application claims priority to Chinese Patent Application No. 202410157141.8, filed to the China National Intellectual Property Administration on January 31, 2024 and entitled "Method and Apparatus for Configuring Test Project", the disclosure of which is hereby incorporated by reference in its entirety.

### Technical Field

The disclosure relates to the technical field of computers, and in particular to a method and apparatus for configuring a test project, a non-transitory computer-readable storage medium, a control terminal and a sample analysis system.

### Background

A biological sample is tested and analyzed outside an organism by an experimental method in in-vitro diagnosis. A sample analysis instrument and a control terminal are typically involved in sample analysis. The control terminal is responsible for information management and experimental control, and the sample analysis instrument is responsible for executing experimental operations.

It is necessary for a user to configure a test project in the control terminal, and relevant experimental programs are configured for the test project. Subsequently, it is further necessary to input sample information and register a project to be tested for a sample to be tested, and then the control terminal controls the sample analysis instrument to execute corresponding experimental operations according to the sample to be tested and the project to be tested.

However, it is generally necessary for an existing method for configuring a test project to create and configure a plurality of test projects in the control terminal for the same test project. As a result, a configuration has a cumbersome operation and low efficiency. Moreover, there will be a heavy workload when a project configuration is modified in a later period. Furthermore, it is easy to cause confusion when the user registers a sample, thereby delaying a progress of experimental test, and even resulting in an adverse consequence caused by wrong registration.

### Summary

In view of that, it is necessary to provide a method and apparatus for configuring a test project, a control terminal and a sample analysis system aiming at the technical problem provided in the prior art that a project configuration has a cumbersome operation, low efficiency, large modification workload and delayed experimental test.

A method for configuring a test project includes:
acquiring a program mapping relation of a target test project, where the program mapping relation includes mapping relations between a plurality of target sample types and at least one target first experimental program;
acquiring program configuration information of a target second experimental program, where the target second experimental program uniquely corresponds to a project type of the target test project; and
configuring the target test project on the basis of the program mapping relation and the program configuration information of the target second experimental program.

An apparatus for configuring a test project includes:
a mapping relation acquisition component, configured to acquire a program mapping relation of a target test project, where the program mapping relation includes mapping relations between a plurality of target sample types and at least one target first experimental program;
a program configuration acquisition component, configured to acquire program configuration information of a target second experimental program, where the target second experimental program uniquely corresponds to a project type of the target test project; and
a test project configuration component, configured to configure the target test project on the basis of the program mapping relation and the program configuration information of the target second experimental program.

A non-transitory computer-readable storage medium stores a computer program, and the computer program causes a processor to execute the steps of the method as described above when executed by the processor.

A control terminal includes a memory and a processor, where the memory stores a computer program, and the computer program causes the processor to execute the method as described above when executed by the processor.

A sample analysis system includes the control terminal as described above a sample analysis instrument, where the control terminal is communicatively connected to the sample analysis instrument;
the control terminal is configured to obtain and store a program mapping relation of a target test project and program configuration information of a target second experimental program, where the program mapping relation includes mapping relations between a plurality of target sample types and at least one target first experimental program, and the target second experimental program uniquely corresponds to a project type of the target test project;
the control terminal is further configured to divide samples to be tested into experimental batches on the basis of sample types of the samples to be tested and projects to be tested, where the samples to be tested in the same experimental batch have the same sample type and the same project to be tested, and alternatively, the samples to be tested in the same experimental batch correspond to the same first experimental program and have the same project to be tested; and send an experimental control instruction to the sample analysis instrument for each experimental batch on the basis of the sample types of the experimental batch, program mapping relations of the projects to be tested of the experimental batch, and program configuration information of a second experimental program; and
the sample analysis instrument is configured to perform corresponding experimental operations on entity samples corresponding to the samples to be tested in each experimental batch on the basis of the experimental control instruction.

According to the method and apparatus for configuring a test project, the computer-readable storage medium, the control terminal and the sample analysis system, the mapping relations between the plurality of sample types corresponding to the target test project and the at least one first experimental program are acquired, the program configuration information of the second experimental program uniquely corresponding to the project types of the target test projects is acquired, and the target test project is configured on the basis of the mapping relations and the program configuration information. Thus, the first experimental programs corresponding to different sample types can be configured for one test project. For a case in which one test project has the plurality of sample types, it is only necessary to create one test project and unnecessary to create one test project for each sample type, thereby simplifying workload of project configuration and later modification, and improving convenience and efficiency of the project configuration. Moreover, only one test project can be displayed for the same test project in the sample registration interface, thereby effectively avoiding confusion when the user registers a sample, and improving registration efficiency and accuracy.

### Brief Description of the Drawings

Fig. 1 is an application environment diagram of the method for configuring the test project in some embodiments of the present disclosure;
Fig. 2 is a schematic flow diagram of the method for configuring the test project in some embodiments of the present disclosure;
Fig. 3 is a diagram showing an operation of configuring a first experimental program for a test project in some embodiments of the present disclosure;
Fig. 4 is a schematic interface diagram of a project configuration interface in some embodiments of the present disclosure;
Fig. 5 is a schematic interface diagram of a project configuration interface in some embodiments of the present disclosure;
Fig. 6 is a schematic diagram of a software interface of control software in some embodiments of the present disclosure;
Fig. 7 is a schematic process diagram showing an example of creating and configuring a nucleic acid extraction program;
Fig. 8 is a schematic process diagram showing an example of creating and configuring a nucleic acid extraction program;
Fig. 9 is a schematic process diagram showing an example of creating and configuring a nucleic acid test project;
Fig. 10 is a schematic process diagram showing an example of creating and configuring a nucleic acid test project;
Fig. 11 is a structural block diagram of the apparatus for configuring the test project in some embodiments of the present disclosure; and
Fig. 12 is a structural block diagram of a control terminal in some embodiments of the present disclosure.

### Detailed Description of the Embodiments

The terms used in the disclosure, "comprise", "include", "have", "display" and any variations thereof, are intended to cover non-exclusive inclusions. For example, a process, a method, a system, a product, or a device that includes a series of steps or units is not necessarily limited to the steps or units explicitly listed, but may include other steps or units not explicitly listed or inherent to the process, method, product, or device. The terms "plurality", "various", and their any variations refer to at least two, unless otherwise specifically limited; and the terms "first", "second", etc. are used to make naming distinctions between similar objects, but these objects themselves are not limited by these terms, and the terms are not to be construed as indicating or implying relative importance or implicitly specifying the number of indicated technical features, a specific order or primary and secondary relations.

In order to make the objectives and advantages of the disclosure clearer, a detailed explanation of the corresponding problem in the prior art found by the inventor is provided:
it is necessary to configure a test project on a control terminal in a process of sample analysis in the field of in-vitro diagnosis, and relevant experimental programs are configured for the test project. Usually, it is necessary to configure various experimental programs for the test project. The experimental programs at least include a first experimental program and a second experimental program. The first experimental program refers to an experimental program relevant to a sample type of the test project, and the second experimental program refers to an experimental program relevant to a project type of the test project. For example, when a nucleic acid test project is configured, it is necessary to configure a nucleic acid extraction program and a nucleic acid amplification program for the nucleic acid test project. The nucleic acid extraction program is relevant to a sample type of the nucleic acid test project, and the nucleic acid amplification program is relevant to a project type of the nucleic acid test project.

However, the inventor finds that a current configuration method is to create a test project in the control terminal, configure the first experimental program corresponding to a sample type for the test project, and configure the second experimental program corresponding to the test project for the test project. However, one test project may correspond to a plurality of sample types, and the plurality of sample types may correspond to a plurality of first experimental programs. To this end, it is necessary to create a plurality of test projects in the control terminal. The first experimental program corresponding to one sample type is configured for each test project. In addition, since the project types of the plurality of test projects are the same (i.e. the same test project), the same second experimental program is configured for each test project. For example, a corona virus disease (COVID) nucleic acid test project corresponds to three sample types of a throat swab, serum and feces, and the three sample types correspond to nucleic acid extraction programs TQ1, TQ2 and TQ3 respectively. Moreover, the test project of COVID nucleic acid test corresponds to a nucleic acid amplification program KZ. The control terminal creates three COVID nucleic acid test projects P1, P2 and P3, the nucleic acid extraction program TQ1 and the nucleic acid amplification program KZ are configured for the COVID nucleic acid test project P1, the nucleic acid extraction program TQ2 and the nucleic acid amplification program KZ are configured for the COVID nucleic acid test project P2, and the nucleic acid extraction program TQ3 and the nucleic acid amplification program KZ are configured for the COVID nucleic acid test project P3.

Thus, it is necessary to create and configure the plurality of test projects for the same test project. The plurality of test projects have a number of common configuration information, and project configuration and later modification have a number of repeated operations, resulting in large configuration workload, cumbersome operation, and low efficiency.

Moreover, the inventor further finds that after the project configuration is completed, the control terminal lists and displays project names of all the created test projects in a sample registration interface when projects to be tested are registered for samples to be tested. A user distinguishes the test projects according to the project names, and further selects the corresponding test projects for each sample to be tested for registration. However, for a case in which the plurality of test projects are created for one test project, the plurality of test projects may be simultaneously displayed in the sample registration interface. For example, the above COVID nucleic acid test projects P1, P2 and P3 are simultaneously displayed.

Thus, a user easily forgets the sample type corresponding to each displayed test project, resulting in confusion about which test project to select for registration for the sample to be tested. For example, when the user selects the project to be tested for a throat swab sample, the user may not be able to distinguish which of P1, P2 and P3 is suitable for the throat swab sample type, which may result in low registration efficiency, thereby delaying an experimental test progress, or result in a registration error, resulting in an adverse consequence.

In order to solve the above problem, the embodiments of the disclosure provide a method and apparatus for configuring a test project, a non-transitory computer-readable storage medium, a control terminal and a sample analysis system. First experimental programs corresponding to different sample types are configured for one test project. For a case in which one test project has the plurality of sample types, it is only necessary to create one test project and unnecessary to create one test project for each sample type, thereby simplifying workload of project configuration and later modification, and improving convenience and efficiency of the project configuration. Moreover, only one test project can be displayed for the same test project in a sample registration interface, thereby effectively avoiding confusion when a user registers a sample, and improving registration efficiency and accuracy.

In order to conveniently understand the technical implementation of the embodiments of the disclosure, the application field and the application environment of the embodiments of the disclosure are described in detail:
the embodiments of the disclosure may be applied to the field of in-vitro diagnosis, and the subdivision field is specifically nucleic acid test, for example.

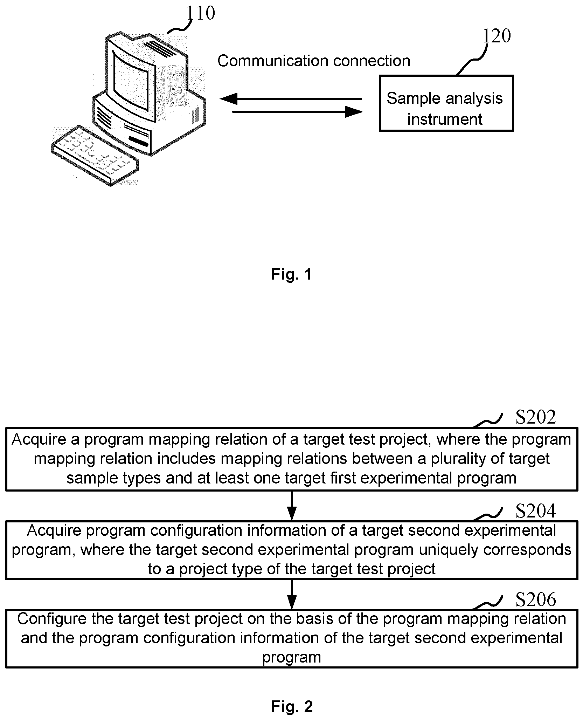

The application environment may be as shown in Fig. 1, and includes a control terminal 110 and a sample analysis instrument 120. Control software is installed on the control terminal 110, and the control terminal 110 is communicatively connected to the sample analysis instrument 120.

In some embodiments of the present disclosure, a test project is configured by means of the control software in the control terminal 110. That is, the control software acquires project configuration information of the test project, the project configuration information includes mapping relations between a plurality of sample types corresponding to the test project and at least one first experimental program, and program configuration information of a second experimental program uniquely corresponding to a project type of the test project. Further, the control software configures the test project on the basis of relevant project configuration information. After a user loads a sample into the sample analysis instrument 120, the user registers the sample in the control software. In some embodiments of the present disclosure, the user inputs sample information of a sample to be tested and register a project to be tested for the sample to be tested, so as to obtain sample registration information. Further, the control software generates an experimental control instruction according to the sample registration information of the sample to be tested and project configuration information of the registered project to be tested, and sends the experimental control instruction to the sample analysis instrument 120.

The sample analysis instrument 120 executes corresponding experimental operations on the sample to be tested according to an experimental operation mode indicated by the experimental control instruction, so as to obtain experimental test data and sends the experimental test data to the control software. Correspondingly, the control software analyzes and processes the experimental test data to obtain an experimental test result, and displays the experimental test result to the user.

The control terminal 110 may be, but not limited to, various desktop computers, notebook computers, tablet computers, Internet of Things devices, and smart phones. The sample analysis instrument 120 is an in vitro diagnostic instrument, and may be, but not limited to, a polymerase chain reaction (PCR) instrument.

It should be noted that a server (not shown) may be further additionally arranged in the application environment of the embodiments of the disclosure, and the control terminal 110 is communicatively connected to the sample analysis instrument 120 and the server separately. The server may be implemented by means of an independent server or a server cluster composed of a plurality of servers. Accordingly, the control software in the control terminal 110 is responsible for providing a human-computer interaction interface to perform relevant operations by the user, and corresponding data processing and storage tasks are implemented by the server. In some embodiments of the present disclosure, for example, the user performs relevant operations in an interface of the control software, the control software obtains the project configuration information of the test project and the sample registration information of the sample to be tested, and sends the information to the server, and further, the server further processes and stores the information. When an experiment is started, the server generates the experimental control instruction according to the relevant information, and sends the experiment control instruction to the sample analysis instrument 120 by means of the control software in the control terminal 110.

It should be further noted that the application environment of the embodiments of the disclosure may also only include an intelligent experimental device. For example, functions of the control terminal 110 and the sample analysis instrument 120 in Fig. 1 are integrated into an integrated intelligent sample analysis instrument. Thus, whole process matters of information management, experimental control and experimental operation execution may be centralized on a single device.

In order to understand the application environment of the embodiments of the disclosure more clearly, the embodiments of the disclosure applied to the subdivision field of nucleic acid test of in-vitro diagnosis will be supplementally described as an example.

The control terminal 110 in the application environment shown in Fig. 1 may be an upper personal computer (PC) on which control software is installed, and the sample analysis instrument 120 may be a PCR integrated machine.

The PCR integrated machine is also called as a nucleic acid integrated machine, and is a PCR instrument. PCR is abbreviation of polymerase chain reaction. The PCR integrated machine includes a reagent preparation component, a nucleic acid extraction component and a nucleic acid amplification component. The reagent preparation component is configured to complete tasks of configuring an extraction plate and an amplification plate. That is, the extraction plate is configured by means of an extraction reagent, and the amplification plate is configured by means of an amplification reagent. The nucleic acid extraction component is configured to complete tasks of sample addition, nucleic acid extraction and nucleic acid transfer. That is, a sample is collected from a sample tube to be added into the extraction plate, nucleic acids are extracted from the sample in the extraction plate, and the extracted nucleic acids are transferred into the amplification plate. The nucleic acid amplification component is configured to amplify nucleic acids in the amplification plate according to a PCR technology to obtain PCR test data.

If it is necessary for the sample to be tested to perform a nucleic acid test project, corresponding nucleic acid test projects may be configured in the control software of the upper PC. That is, the control software acquires project configuration information of the nucleic acid test project, the project configuration information includes mapping relations between a plurality of sample types corresponding to the nucleic acid test project and at least one nucleic acid extraction program, and program configuration information of a nucleic acid amplification program corresponding to the nucleic acid test project. Moreover, the control software configures the nucleic acid test project on the basis of the relevant project configuration information. After the user loads the sample into a sample bin of the PCR integrated machine, the user registers the sample in the control software. In some embodiments of the present disclosure, the user inputs sample information of the sample to be tested and registers the configured nucleic acid test project as a project to be tested of the sample to be tested, so as to obtain sample registration information. Further, the control software generates an experimental control instruction according to the sample registration information of the sample to be tested and the project configuration information of the registered project to be tested, and sends the experimental control instruction to the PCR integrated machine.

The PCR integrated machine correspondingly completes experimental operations of configuring the extraction plate and the amplification plate, adding the sample into the extraction plate, extracting the nucleic acids, transferring the nucleic acids and amplifying the nucleic acids according to an experimental operation mode indicated by the experimental control instruction, so as to obtain PCR test data, and sends the PCR test data to the control software. Further, the control software analyzes and processes the PCR test data to obtain an experimental test result of the sample to be tested, and displays the experimental test result in a relevant interface.

The embodiments of the disclosure will be further described in detail below in combination with the accompanying drawings and the examples. It should be noted that the method according to the embodiments of the disclosure applied to the control terminal in Fig. 1 will be described as an example below, but is only an exemplary description and not limited to being executed only by the control terminal.

In some embodiments of the present disclosure, as shown in Fig. 2, a method for configuring a test project is provided. The method may includes following S202-S206.

S202, a program mapping relation of a target test project is acquired.

A test project refers to a medical project in which a biological sample is tested and analyzed to provide a test result. The biological sample is body fluid or tissue collected and used for test and analysis, such as blood, saliva, urine, skin, or other tissue. Corresponding to different sample analysis instruments, the test project may be a nucleic acid test project, such as a COVID nucleic acid test project and a human papilloma virus (HPV) nucleic acid test project.

The target test project is a test project to be configured. The target test project may be determined on the basis of an operation by a user. For example, a user triggers control software to create a new test project, and the control software uses the newly created test project as the target test project. For another example, the control software displays created test projects, the user selects a test project from the created test projects, and the control software uses the selected test project as the target test project.

In some embodiments, it is necessary to configure an experimental program for the target test project, and the configured experimental program includes a first experimental program. The experimental program refers to an operation flow of an experimental link involved in the test project. The first experimental program refers to an experimental program relevant to a sample type of a test project. For example, for the nucleic acid test project, the first experimental program includes a nucleic acid extraction program. The nucleic acid extraction program refers to an operation flow of a nucleic acid extraction link in the nucleic acid test project, generally includes a fluid transferring step of and an extraction step, and is relevant to a sample type of the nucleic acid test project.

The program mapping relation and the test project have a one-to-one relation. That is, one test project corresponds to a set of program mapping relations. The program mapping relation includes mapping relations between a plurality of sample types of the test project and at least one first experimental program. Correspondingly, the program mapping relation of the target test project includes mapping relations between a plurality of target sample types and at least one target first experimental program. The target sample types are sample types corresponding to the target test project, and the target first experimental program is the first experimental program corresponding to the target test project. The program mapping relation may be implemented by means of a common data structure, such as a mapping table, at a data processing level.

In some embodiments, for one target test project, the program mapping relation of the target test project may have three cases:
case 1, the program mapping relation may include one-to-one mapping relations between the plurality of target sample types and a plurality of target first experimental programs. Different target sample types correspond to different target first experimental programs. For example, for a target test project A1, the program mapping relation includes mapping relations between five target sample types and five target first experimental programs. The five target sample types correspond to the five target first experimental programs one to one. That is, the five different target sample types correspond to the five different target first experimental programs.
Case 2, the program mapping relation may include mapping relations between the plurality of target sample types and the plurality of target first experimental programs, but the mapping relations are not one-to-one relations, but the number of the target sample types is greater than that of the target first experimental programs. Each target first experimental program corresponds to one or more target sample types, but at least one target first experimental program simultaneously corresponds to the plurality of target sample types. That is, in the plurality of target sample types, each sample type corresponds to one target first experimental program, but part of the target sample types correspond to the same target first experimental program. For example, for a target test project A2, the program mapping relation includes mapping relations between five target sample types and four target first experimental programs. Two target sample types correspond to the same target first experimental program, and the other three target sample types correspond to the other three target first experimental programs one to one. Alternatively, for a target test project A3, the program mapping relation includes mapping relations between five target sample types and three target first experimental programs. Two target sample types correspond to the same target first experimental program, the other two target sample types correspond to another target first experimental program, and the remaining one target sample type corresponds to the remaining one target first experimental program.
Case 3, the program mapping relation may include mapping relations between the plurality of target sample types and one target first experimental program, and all the target sample types correspond to the same target first experimental program. For example, for a target test project A4, the program mapping relation includes mapping relations between two target sample types and one target first experimental program, and both of the two sample types correspond to the same target first experimental program.

In addition, the program mapping relation of the target test project has various sources. For example, the program mapping relation may be obtained on the basis of an input operation by the user in a relevant interface of the control software, or the program mapping relation may be obtained by scanning a code, or the program mapping relation may be transmitted to the control terminal by other devices. Correspondingly, a specific mode of acquiring the program mapping relation of the target test project may be as follows: a mapping relation between a target sample type and a corresponding target first experimental program is obtained, then a mapping relation between a next target sample type and a corresponding target first experimental program is obtained, and so on, until mapping relations between all target sample types of the target test project and all target first experimental programs are obtained. So far, the program mapping relation of the target test project is obtained. Alternatively, packaged data may be directly obtained. The packaged data includes mapping relations between all target sample types of the target test project and all target first experimental programs. That is, the packaged data includes the program mapping relation of the target test project.

S204, program configuration information of a target second experimental program is acquired.

In some embodiments, it is further necessary to configure a second experimental program in addition to configuring the first experimental program for the target test project. The second experimental program refers to an experimental program relevant to a project type of the test project, and different types of test projects may correspond to different second experimental programs. For example, for the nucleic acid test project, the second experimental program may include a nucleic acid amplification program. The COVID nucleic acid test project and the HPV nucleic acid test project may correspond to different nucleic acid amplification programs, but as long as the test project is the COVID nucleic acid test project, the nucleic acid amplification program is the same regardless of which the sample type is a throat swab, serum or feces.

Correspondingly, the target second experimental program refers to the second experimental program corresponding to the target test project, and the target second experimental program uniquely corresponds to the project type of the target test project. Thus, it may be understood that in some embodiments, for one target test project, one or more first experimental programs may be configured for the target test project corresponding to the plurality of sample types of the target test project. However, since the project type of the target test project is determined and unique, only one second experimental program may be configured for the target test project.

The program configuration information is information configured to configure an experimental program. Contents included in the program configuration information may be set according to test requirements. For example, the program configuration information of the second experimental program usually includes experimental operation information. The experimental operation information is configured to indicate experimental operation modes of corresponding experimental links. That is, the sample analysis instrument may execute experimental operations according to the experimental operation modes indicated by the corresponding experimental operation information in the experiment links. For example, for the nucleic acid test project, experimental operation information of the nucleic acid amplification program may include program segment distribution information and specific setting information of program segments. The program segment distribution information includes the number of constant temperature segments, cycle segments and melting segments, and sequential relations of the program segments, for example. The specific setting information of the program segments include temperature, time and cycle times of the constant temperature segments, for example. The control software may control the sample analysis instrument to perform nucleic acid amplification processing according to the experimental operation modes indicated by the experimental operation information. In addition, the program configuration information of the second experimental program may further include basic program information. The basic program information is configured to describe basic situations of the second experimental program, such as a program identifier. The program identifier is a unique identifier of the second experimental program. In some embodiments of the present disclosure, the control software may generate a program ID for the second experimental program as the program identifier, or the control software may acquire relevant information (for example, program name) of the second experimental program as the program identifier.

Similarly, the program configuration information of the target second experimental program may also have various sources. For example, the program configuration information of the target second experimental program may be obtained on the basis of an input operation by the user in a relevant interface of the control software, or the program configuration information of the target second experimental program may be obtained by scanning a code, or the program configuration information of the target second experimental program may be transmitted to the control terminal by other devices.

S206, the target test project is configured on the basis of the program mapping relation and the program configuration information of the target second experimental program.

In some embodiments, in a process of configuring the target test project, a corresponding experimental program may be configured for the target test project on the basis of the program mapping relation of the target test project and the program configuration information of the target second experimental program. That is, the first experimental program is configured for the target test project on the basis of the program mapping relation of the target test project, and the second experimental program may be configured for the target test project on the basis of the program configuration information of the target second experimental program.

It should be noted that configuring the first experimental program for the target test project may be that a test project is created, but a first experimental program has not been configured for the test project, subsequently, the test project is used as the target test project to obtain the program mapping relation of the target test project, and then the first experimental program is configured for the target test project from scratch according to the program mapping relation. In other embodiments, configuring a first experimental program for a target test project may be that a test project is created, a first experimental program has been configured for the test project, but subsequently, it is necessary to modify the first experimental program for the test project, such that the test project is used as the target test project to obtain a program mapping relation of the target test project, and the first experimental program is updated and configured for the target test project according to the program mapping relation. The second experimental program is configured for the target test project in the same way, which will not be described herein.

In some embodiments of the present disclosure, an embodiment of configuring the first experimental program and the second experimental program for the target test project may be that the program mapping relation of the target test project and a project identifier of the target test project are associatively stored, and the program configuration information of the target second experimental program and the project identifier of the target test project are associatively stored. The project identifier is a unique identifier of the target test project. In some embodiments of the present disclosure, the control software may generate a project ID for the target test project as the project identifier, or the control software may acquire relevant information (for example, project name) of the target test project as the project identifier.

For ease of understanding, an exemplary description is provided below in combination with a particular embodiment. Control software generates a project mapping table in advance. The table includes fields of a project identifier, a sample type, a program identifier of a first experimental program and a program identifier of a second experimental program. For a target test project, the control software obtains a project identifier and a program mapping relation of the target test project, and program configuration information of a second experimental program. The program mapping relation includes type identifiers of a plurality of sample types of the target test project and a program identifier of a first experimental program corresponding to each sample type. The program configuration information of the second experimental program includes a program identifier of the second experimental program. Obtained information of the project identifier, the plurality of type identifiers, the program identifier of the at least one first experimental program, and the program identifier of the second experimental program is correspondingly stored into the project mapping table, and the project mapping table is stored in a database. That is, the first experimental program and the second experimental program are configured for the target test project. In addition, configuring the first experimental program and the second experimental program for other target test projects is also that the above information of the target test project is saved into the project mapping table, and the project mapping table records relevant configuration information of all the test projects created in the control software according to preset fields.

It should be noted that when the target test project is configured, configuration items involved may be determined according to an actual project situation. Other configuration items may be further configured for the target test project in addition to configuring the first experimental program and the second experimental program for the target test project. For example, when the nucleic acid test project is configured, five configuration items of basic project information, a reagent component, a test target, a result determination and a quality control setting may be further configured for the nucleic acid test project generally in addition to configuring the extraction program and the amplification program for the nucleic acid test project. Thus, relevant configuration information of other configuration items may be further acquired in addition to acquiring the program mapping relation of the target test project and the program configuration information of the target second experimental program. Accordingly, corresponding configuration items are configured for the target test project.

According to the method for configuring a test project, the mapping relations between the plurality of sample types corresponding to the target test project and the at least one first experimental program is acquired, the program configuration information of the second experimental program uniquely corresponding to the project type of the target test project is acquired, and the target test project is configured on the basis of the mapping relations and the program configuration information. Thus, the first experimental programs corresponding to different sample types can be configured for one test project. For a case in which one test project has the plurality of sample types, it is only necessary to create one test project and unnecessary to create one test project for each sample type, thereby simplifying workload of project configuration and later modification, and improving convenience and efficiency of the project configuration. Moreover, only one test project can be displayed for the same test project in the sample registration interface, thereby effectively avoiding confusion when the user registers a sample, and improving registration efficiency and accuracy.

In some embodiments of the present disclosure, the step of acquiring a program mapping relation of a target test project, i.e. S202, may include: a plurality of target sample types are determined on the basis of a sample type input operation by a user in a project configuration interface; a first experimental program associated with the target sample type is determined for each target sample type on the basis of program configuration information of first experimental programs stored in advance, and a target first experimental program corresponding to the target sample type is determined from the associated first experimental program on the basis of a program selection operation by a user in a project configuration interface; and the program mapping relation of the target test project is obtained on the basis of the plurality of target sample types determined and the target first experimental programs corresponding to the target sample types.

The project configuration interface is a software interaction interface configured to implement project configuration. The project configuration interface may be implemented in various ways, such as a single page or multiple pages. For example, the project configuration interface may use tabs. That is, the project configuration interface includes a plurality of tab options corresponding to a plurality of configuration items of the target test project. Any tab option is selected to display the configuration page of the corresponding configuration item. The tab options include a tab option corresponding to a configuration item of the first experimental program. The tab option is selected to correspondingly display a configuration page of the first experimental program, so as to perform relevant configuration operations by the user, thereby configuring the first experimental program for the target test project.

The sample type input operation refers to an operation of inputting the sample type for the target test project by the user in the project configuration interface, i.e. an operation of inputting a target sample type. An embodiment of the sample type input operation is, for example, that the user selects one sample type from the sample types displayed in a drop-down box. For another example, the user fills in relevant information of the target sample type in a text control.

The program selection operation refers to an operation of inputting the first experimental program for the target test project by the user in the project configuration interface, i.e. an operation of selecting the target first experimental program corresponding to the target sample type for the target sample type. An embodiment of the program selection operation is, for example, that the user selects one first experimental program from first experimental programs displayed in the drop-down box.

In some embodiments, for any target sample type input by the user, the first experimental program associated with the target sample type is determined on the basis of program configuration information of first experimental programs stored in advance. Thus, it may be understood that before the first experimental program associated with the target sample type is determined, it is necessary to configure a program for the first experimental program configured for the target test project in advance, i.e. to complete a program configuration task of the target first experimental program in advance, so as to obtain and store the program configuration information of the target first experimental program. Moreover, the program configuration information of the target first experimental program at least includes the sample types associated with the target first experimental program. Following the above example, a COVID nucleic acid test project corresponds to three sample types of a throat swab, serum and feces. Before nucleic acid extraction programs are configured for the COVID nucleic acid test project, it is necessary to configure programs for the three nucleic acid extraction programs TQ1, TQ2 and TQ3, so as to obtain and store the program configuration information of each of the three nucleic acid extraction programs, and to correspondingly record the sample types of the throat swab, the serum and the feces associated with the three nucleic acid extraction programs on the basis of the program configuration information of TQ1, TQ2 and TQ3.

It should be noted that for any target sample type, there may be only one or more determined first experimental programs associated with the target sample type on the basis of the program configuration information of the first experimental programs stored in advance. In practical application, a plurality of target test projects are usually sequentially configured in the control software. Thus, program configuration information of a plurality of first experimental programs configured for a plurality of test projects is simultaneously stored in a database. For any target sample type, if the program configuration information of the plurality of first experimental programs currently stored in the database records the target sample type associated with the first experimental programs, the plurality of first experimental programs determined and associated with the target sample type may appear, and it is necessary for the user to determine the first experimental programs and select one first experimental program from the first experimental programs. But if the program configuration information of only one first experimental program in the database records the target sample type associated with the first experimental program, there is only one determined first experimental program associated with the target sample type, which may be confirmed by the user.

It should be further noted that it is necessary for the user to input the plurality of sample types for the target test project, and to select the first experimental program corresponding to each sample type for each sample type. There are various particular embodiments. For example, the user may sequentially perform operations of inputting one sample type for the target test project, then selecting the corresponding first experimental program for the sample type, then inputting a next sample type, and selecting the corresponding first experimental program for the sample type, and so on, until all the sample types are input for the target test project and the corresponding first experimental program is selected for each sample type. For another example, the user may also input all the sample types of the target test project at one time, and then select the corresponding first experimental program for each sample type.

For ease of a clear understanding, a process of obtaining the program mapping relation in some embodiments will be described below in combination with a specific example:
for a target test project, it is necessary for control software to configure a program for a first experimental program configured for the target test project in advance before the first experimental program is configured for the target test project, i.e. to configure the program for the target first experimental program in advance, so as to obtain and store program configuration information of the target first experimental program. The program configuration information includes a type identifier of a sample type associated with the first experimental program.

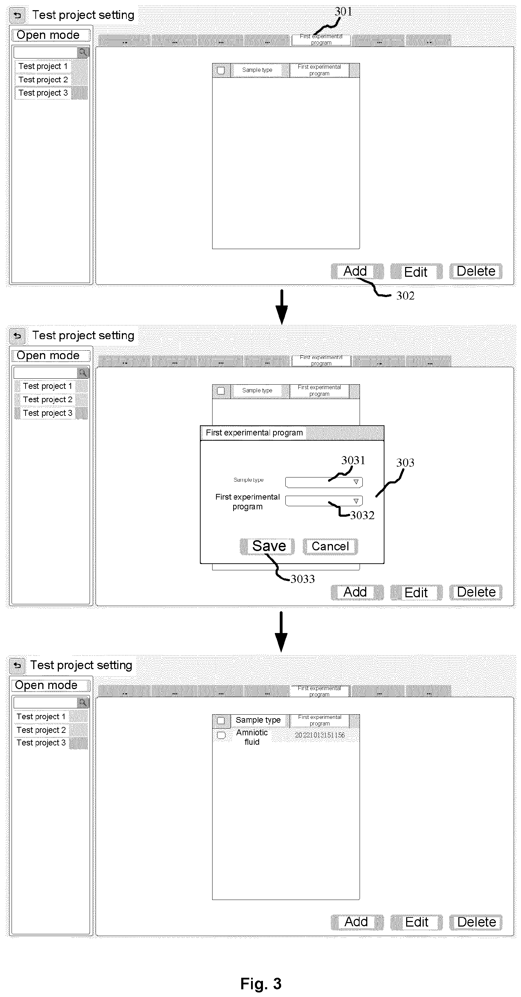

Subsequently, the control software displays a project configuration interface shown in Fig. 3, a tab option 301 corresponding to a configuration item of the first experimental program is displayed in the project configuration interface, a user selects the tab option 301, and the control software displays a configuration page of the first experimental program. The user performs a sample type input operation in the configuration page, and inputs a target sample type. In some embodiments of the present disclosure, the user clicks an add button 302 in the configuration page, a setting pop-up box 303 pops up in the configuration page, and a sample drop-down box 3031 and a program drop-down box 3032 are displayed in the setting pop-up box. The user selects one sample type from sample types displayed in the sample drop-down box 3031, and the selected sample type is a target sample type to be input. The control software acquires a type identifier of the target sample type. Further, the control software determines a first experimental program associated with the target sample type on the basis of the type identifier and program configuration information of first experimental programs stored in a database in advance, and displays the first experimental program in the program drop-down box 3032. The user performs a program selection operation, and selects one first experimental program from the first experimental programs displayed in the program drop-down box. The control software uses the selected first experimental program as the target first experimental program corresponding to the target sample type, and obtains a program identifier of the target first experimental program. Subsequently, the user clicks a save button 3033 in the setting pop-up box, and the control software binds the type identifier of the target sample type with the program identifier of the target first experimental program to obtain a corresponding relation between the target sample type and the target first experimental program, and displays the input target sample type and the target first experimental program corresponding to the target sample type in the configuration page. For example, the user selects the sample type of amniotic fluid and the first experimental program numbered 20221013151156. Fig. 3 correspondingly displays the amniotic fluid and 20221013151156. The above operations are repeated until the user input all the target sample types and selects the target first experimental program corresponding to each target sample type for each target sample type, and the control software correspondingly obtains the type identifiers of the plurality of target sample types, the program identifier of the at least one target first experimental program, and the corresponding relations between the target sample types and the target first experimental program, i.e. obtains the program mapping relation of the target test project.

It should be further noted that in some embodiments, not only the sample input operation and the program selection operation may be performed in the project configuration interface to configure the first experimental program for the target test project, but also relevant configuration operations may be further carried out in the project configuration interface to configure the second experimental program and other configuration items for the target test project. The particular embodiment may use any applicable mode in the art, which is not described herein.

In summary, the example configures the target test project on the basis of the operation by the user in the project configuration interface, supports the user to flexibly input the project configuration information, has high compatibility with project configuration of a third-party manufacturer, and has convenient operation.

In some embodiments of the present disclosure, before the step of acquiring a program mapping relation of a target test project, i.e. before S202, the method further may further include: basic project information of the target test project is acquired on the basis of a basic information input operation by the user in the project configuration interface; and the target test project is created on the basis of the basic project information in response to a project creation operation by the user. On this basis, the step of configuring the target test project on the basis of the program mapping relation and the program configuration information of the target second experimental program. i.e. S206 may include: the target test project is updated on the basis of the program mapping relation and the program configuration information of the target second experimental program.

The basic information input operation refers to an operation of inputting basic project information of the target test project for the target test project by the user in the project configuration interface. The basic project information is configured to describe basic situations of the target test project, such as a project name and a project type. An embodiment of the basic information input operation is, for example, that the user fills in the basic project information in a text control, the user selects the basic project information in a drop-down box, or the two modes are combined.

The project creation operation refers to an operation of triggering creation of the target test project. That is, the project creation operation of the user may trigger control software to create the target test project. An embodiment of the project creation operation is, for example, that for a click operation on a project save button in the project configuration interface or a preset gesture operation by the user in the project configuration interface, in some embodiments of the present disclosure, the user draws a preset track in the project configuration interface by means of a touch mode or a mouse operation mode.

In some embodiments of the present disclosure, the user performs the basic information input operation in the project configuration interface, then the control software obtains the basic project information of the target test project, and further, the user performs the project creation operation. The control software creates the target test project on the basis of the basic project information in response to the operation. So far, the control software creates one target test project, but only the basic project information is configured for the target test project, and other configuration items such as a first experimental program and a second experimental program, have not been configured for the target test project. Subsequently, the user performs the sample type input operation and the program selection operation for the target test project, and the control software obtains the program mapping relation of the target test project, configures the first experimental program for the target test project on the basis of the program mapping relation, obtains program configuration information of the target second experimental program, and configures the second experimental program for the target test project on the basis of the program configuration information, thereby updating relevant configurations of the target test project.

In summary, in some embodiments, the user only inputs the basic project information of the target test project, such that the control software may be triggered to create the target test project. Subsequently, an opportunity is selected to further configure other configuration items such as the experimental program for the target test project according to an actual situation. It is not required that the user inputs the relevant configuration information of all the configuration items at one time, such that flexibility of the project configuration is high, and the requirements of the user in different scenarios may be better satisfied.

It should be noted that in other examples, a target test project may be allowed to be created only after the user inputs relevant configuration information of all configuration items of the target test project. Although the mode requires the user to input a number of information at one time and an operation pressure is high, project configuration contents may be prevented from missing, and an adverse effect may be avoided. For example, it is found that project configuration contents are missing when experimental test is started, and rework is required, thereby greatly affecting an experimental process.

In some embodiments of the present disclosure the step of acquiring a program mapping relation of a target test project, i.e. S202, may include: project graphic code information is analyzed to obtain the program mapping relation of the target test project, where the project graphic code information is obtained by means of scanning by a code scanning apparatus.

A project graphic code is a graphic code for configuring the target test project, and the project graphic code and the target test project have a one-to-one relation. That is, one target test project corresponds to one project graphic code. The project graphic code may be specifically a two-dimensional code or a bar code, and may seal relevant project configuration information. In practical application, the project graphic code is usually printed on a kit.

The code scanning apparatus is an apparatus for scanning the project graphic code, and specifically may be a code scanning gun, a tablet, a mobile phone or any other applicable device. In addition, the code scanning apparatus establishes a communication connection to a control terminal in a wired or wireless mode.

In some embodiments, the user scans the project graphic code by means of the code scanning apparatus, and the control software obtains the project graphic code information, and analyzes the project graphic code information according to a preset decoding program to obtain the program mapping relation of the target test project. In some embodiments of the present disclosure, the control software analyzes the project graphic code information to obtain type identifiers of a plurality of target sample types, a program identifier of at least one target first experimental program, and corresponding relations between the type identifiers and the program identifier.

In summary, in some embodiments, the program mapping relation of the target test project may be obtained by scanning the code. It is unnecessary for the user to manually input a large amount of information, such that input of the project configuration information may be efficiently and conveniently completed. Moreover, the project configuration information is encapsulated in the graphic code in advance, and information contents are not disclosed to the outside, thereby being conducive to information confidentiality for a self-developed and self-produced scenario.

In some embodiments of the present disclosure, before the step of configuring the target test project on the basis of the program mapping relation and the program configuration information of the target second experimental program, i.e. before S206, the method may further include: project graphic code information is analyzed to obtain basic project information of the target test project. On this basis, the step of acquiring program configuration information of a target second experimental program, i.e. S204, may include: the project graphic code information is analyzed to obtain the program configuration information of the target second experimental program; and the step of configuring the target test project on the basis of the program mapping relation and the program configuration information of the target second experimental program, i.e. S206, may include: the target test project is created and configured on the basis of the basic project information, the program mapping relation and the program configuration information of the target second experimental program.

In some embodiments, after the control software analyzes the project graphic code information according to the preset decoding program, the control software may further obtain the basic project information of the target test project and the program configuration information of the second experimental program in addition to obtaining the program mapping relation of the target test project, so as to further create the target test project, and configures the basic project information, the first experimental program, and the second experimental program for the target test project. More project configuration information of the target test project is obtained by scanning the project graphic code, such that the project may be efficiently and conveniently configured.

It should be noted that in other examples, relevant configuration information of all configuration items of the target test project may also be obtained by scanning the project graphic code, so as to further create the target test project, and all the configuration items are configured for the target test project. For example, as described above, for a nucleic acid test project, it is necessary to configure seven configuration items of basic project information, a reagent component, a test target, result determination, a nucleic acid extraction program, a nucleic acid amplification program, and a quality control setting for the nucleic acid test project. To this end, relevant configuration information of the seven configuration items may be encapsulated in the project graphic code. The user scans the project graphic code by means of the code scanning apparatus, and the control terminal analyzes the project graphic code information according to the preset decoding program, such that the configuration information corresponding to each of the seven configuration items may be obtained. Accordingly, the tasks of creating and configuring the corresponding nucleic acid test project may be efficiently completed.

In some embodiments of the present disclosure, a method for configuring a test project according to the embodiments of the disclosure may further include:
a project configuration interface is displayed, where the project configuration interface includes a mode selection control, a project list area and a project editing area, where the mode selection control includes an open option and a closed option;
open test projects are displayed in the project list area when the mode selection control corresponds to the open option, where the open test projects include test projects created on the basis of an operation by the user in the project configuration interface; a configuration page corresponding to a selected open test project is displayed in the project editing area on the basis of a selection operation for the displayed open test projects, where the configuration page corresponding to the open test project includes a configuration page of the first experimental program and a configuration page of the second experimental program; and
closed test projects are displayed in the project list area when the mode selection control corresponds to the closed option, where the closed test projects include test projects created by means of project graphic codes; and a configuration page corresponding to a selected closed test project is displayed in the project editing area on the basis of a selection operation for the displayed closed test projects, where the configuration page corresponding to the closed test project does not include a configuration page of the first experimental program and a configuration page of the second experimental program.

The mode selection control is configured to switch the open option and the closed option, and the user may select the open option and the closed option according to actual requirements. A drop-down box or any other applicable display control may be used in particular implementation. The project list area is an area for displaying created test projects, and the project editing area is an area for displaying configuration pages corresponding to test projects. In some embodiments of the present disclosure, if one test project is selected from the test projects displayed in the project list area, the configuration page corresponding to the selected test project may be correspondingly displayed in the project editing area. Configuration positions and sizes of the mode selection control, the project list area and the project editing area have various embodiments in the project configuration interface, and may be flexibly designed according to actual requirements. For example, the project list area and the project editing area are arranged on a left side and a right side of the project configuration interface respectively, and the mode selection control is arranged above the project list area.

In some embodiments of the present disclosure, the project list area centrally displays all the created closed test projects when the mode selection control corresponds to the closed option. The closed test projects include test projects created by means of project graphic codes, and a specific display mode is, for example, that each closed test project is listed in a form of a list. Thus, the user may understand an overall situation of the closed test projects by means of the project list area, for example, the total number of projects, and may select a specific closed test project in the project list area to further view and edit relevant contents.

The user selects one closed test project from the closed test projects displayed in the project list area, and the configuration page corresponding to the selected closed test project is displayed in the project editing area. In some embodiments of the present disclosure, the configuration page corresponds to a public configuration item of the closed test project, and the configuration pages may correspond to the public configuration items one to one. The public configuration item of the closed test project may be preset according to actual requirements, and one or more public configuration items may be set. However, due to consideration of information confidentiality, the public configuration item set for the closed test project is usually few. For example, the public configuration item is preset to only include the configuration item of basic project information. Thus, for the closed test project, only the configuration page of the basic project information is displayed in the project editing area, and the configuration pages of other configuration items are not displayed. However, it should be noted that in some embodiments, it is required that the public configuration item of the closed test project does not include the two configuration items of the first experimental program and the second experimental program. That is, for the closed test project, a configuration page of the first experimental program and a configuration page of the second experimental program are not displayed in the project editing area.

Contents specifically displayed in the configuration page usually include public configuration information of the corresponding public configuration items, i.e. configuration information disclosed to the outside, and the public configuration information may also be preset according to actual requirements, and specifically may include editing permission information and editing prohibition information. In the configuration page, the editing prohibition information is in a non-editable state, and may be viewed but not edited by the user, while the editing permission information is in an editable state, and may be viewed and edited by the user.

For example, a sample analysis instrument is a PCR integrated machine. Correspondingly, a nucleic acid test project is configured in control software. The public configuration item of the closed test project may be preset to include only the two configuration items of the project basic information and the quality control setting, and not to include five configuration items of the reagent component, the test target, the result determination, the nucleic acid extraction program and the nucleic acid amplification program. Moreover, the public configuration information in the public configuration item of the basic project information is preset to include a project abbreviation, a project name, a project type, a project number, a laboratory information system (LIS) channel number, repetition times, a result unit and a display order. The public configuration information of the quality control setting is preset to include a quality control type required for each batch of experiments. The LIS channel number, the repetition times, the result unit, the display order and the quality control type are preset as editing permission information, and the remaining public configuration information is preset as editing prohibition information.

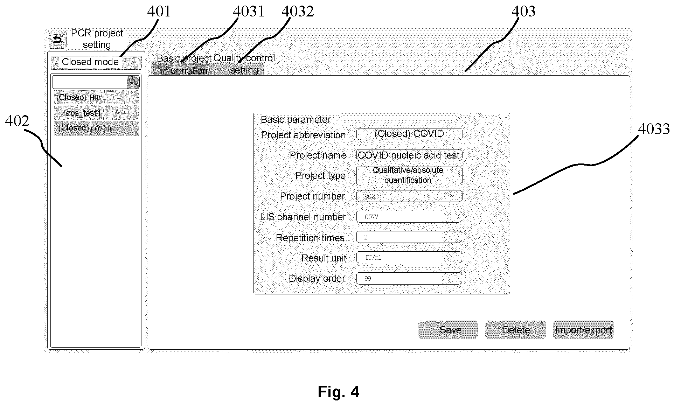

It should be noted that there may be various embodiments for displaying the configuration page in the project editing area. For example, the project editing area displays a tab option corresponding to each public configuration item by means of tabs. Any tab option is selected, and the project editing area displays the configuration page of the corresponding public configuration item. Subsequently, another tab option is selected, and the project editing area is switched to display the configuration page of another public configuration item. Following the above example, for a nucleic acid test project, a project configuration interface is as shown in Fig. 4. When a closed option is selected in a mode selection control 401, a project list area 402 displays all the created closed nucleic acid test projects, the user selects the closed nucleic acid test project of "(closed) COVID" from the closed nucleic acid test projects, and the project editing area 403 displays tab options 4031 and 4032 corresponding to the two public configuration items of the basic project information and the quality control setting respectively. Moreover, control software selects the tab option 4031 corresponding to the basic project information by default, and thus the project editing area displays the configuration page 4033 of the basic project information of the closed nucleic acid test project by default. Subsequently, the user selects the tab option 4032 corresponding to the quality control setting instead, and the project editing area is switched to display the configuration page (not shown) of the quality control setting of the closed nucleic acid test project.

When the mode selection control corresponds to the open option, the project list area centrally displays all the created open test projects. The open test projects include test projects created on the basis of an operation by the user in the project configuration interface. Similarly, a specific display mode is, for example, that each open test project is listed in a form of a list, and the user may understand the overall situation of the open test projects by means of the project list area, and may create an open test project in the project configuration interface and configure relevant contents for the open test project, or directly selects a specific open test project from the project list area to further view and configure relevant contents.

Similar to the closed test project, the user selects one open test project from the open test projects displayed in the project list area, and the project editing area displays the configuration page corresponding to the selected open test project. In some embodiments of the present disclosure, the configuration page corresponds to the public configuration item of the open test project, and one configuration page may correspond to one public configuration item. The public configuration item of the open test project may also be preset according to actual requirements, and a plurality of configuration items are usually set, such that the user completely inputs relevant configuration information of the relevant configuration items to ensure that experimental test is carried out normally. In some embodiments, it is required that the public configuration item of the open test project includes two configuration items of the first experimental program and the second experimental program. That is, for the open test project, the project editing area displays the configuration page of the first experimental program and the configuration page of the second experimental program. Moreover, similarly, the configuration page specifically displays the public configuration information of the corresponding public configuration items. The public configuration information may also be preset according to actual requirements, and specifically includes editing permission information and editing prohibition information, which is not described herein.

Similarly, the nucleic acid test project will be described as an example. The public configuration items of the preset open test project are preset to include seven configuration items of basic project information, a reagent component, a test target, result determination, a nucleic acid extraction program, a nucleic acid amplification program, and a quality control setting. The public configuration information of the basic project information is preset to include a project abbreviation, a project name, a project type, a project number, an LIS channel number, repetition times, a result unit, a display order, a nucleic acid sample addition amount, and an exogenous internal standard sample addition amount. The remaining public configuration information is set as editing permission information in addition to setting the project type as editing prohibition information. The public configuration information of the nucleic acid extraction program is preset to include the sample type and the nucleic acid extraction program, which are set as editing permission information. The public configuration information of the nucleic acid amplification program is preset to include program segment distribution information and specific program segment setting information, which are set as editing permission information. Public configuration information of other configuration items is also set according to actual requirements, but since the public configuration information of other configuration items is not key contents of the embodiments of the disclosure, which is not repeated herein.

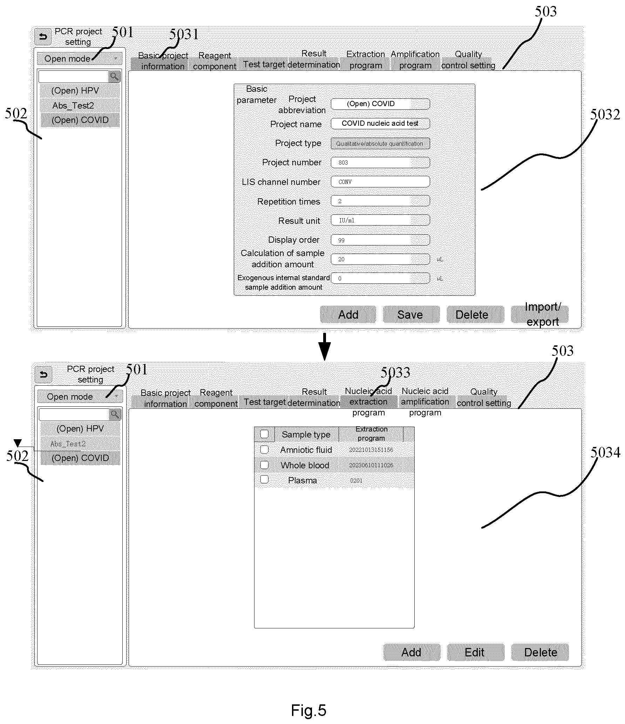

In addition, the embodiment of displaying the configuration page corresponding to the open test project in the project editing area is the same as that of the closed test project above, which is not described herein. Following the above example, tabs are used. As shown in Fig. 5, when an open option is selected in a mode selection control 501, a project list area 502 displays all created open nucleic acid test projects, a user selects the open nucleic acid test project of "(open) COVID" from the open nucleic acid test projects, and the project editing area 503 displays tab options corresponding to seven configuration items of basic project information, a reagent component, a test target, result determination, a nucleic acid extraction program, a nucleic acid amplification program and a quality control setting respectively. Moreover, control software selects a tab option 5031 corresponding to the basic project information by default, and thus a project editing area displays a configuration page 5032 of the basic project information of the open nucleic acid test project by default. Subsequently, the user selects a tab option 5033 corresponding to a nucleic acid extraction program instead, and the project editing area is switched to display a configuration page 5034 of the nucleic acid extraction program of the open nucleic acid test project.

In summary, in some embodiments, two information input modes of a closed mode and an open mode are simultaneously supported. In the open mode, the target test project is created and configured on the basis of the operation by the user in the project configuration interface, and the user inputs and views relevant project configuration information in the project configuration interface. In the closed mode, the target test project is created and configured by scanning the code, and further, the user views and edits a small amount of project configuration information in the project configuration interface. Thus, the example may be flexibly adapted to project configuration requirements under different scenarios. The project may be efficiently, conveniently and safely configured for the self-developed and self-produced scenario. The example may also be well compatible and the project configuration information is flexibly input for a scenario of the third-party manufacturer.

In some embodiments of the present disclosure, a method for configuring a test project according to the embodiments of the disclosure may include at least one of the following items: item 1: program configuration information of a target first experimental program is obtained and stored on the basis of a program information input operation by a user in a program configuration interface; and item 2: program graphic code information is analyzed to obtain and store program configuration information of a target first experimental program.

As described above, it is necessary to configure a program for the target first experimental program, so as to obtain and store the program configuration information of the target first experimental program. Similar to configuration of the target test project, the target first experimental program may be created and configured on the basis of the operation by the user in the program configuration interface, the target first experimental program may be created and configured by scanning the code, or both of the two modes may be supported.

In some embodiments of the present disclosure, program configuration information of a target first experimental program is obtained and stored on the basis of a program information input operation by a user in a program configuration interface.

The program configuration interface is a software interaction interface for configuring a first experimental program. Similar to a project configuration interface, the program configuration interface may also use an embodiment of a single page or multiple pages, and specifically uses tabs, for example. The program information input operation refers to an operation of inputting the program configuration information for the target first experimental program in the program configuration interface. A particular example is, for example, that the user fills in the program configuration information in a text control, the user selects the program configuration information in a drop-down box, or the two modes are combined. The program configuration information of the first experimental program includes basic program information and experimental step information. The basic program information is configured to describe a basic situation of the first experimental program, at least includes the sample type associated with the first experimental program, and usually further includes a program name; and the experimental step information is configured to indicate experimental operation modes of corresponding experimental links. For example, for a nucleic acid test project, experimental operation information of a nucleic acid extraction program of the nucleic acid test project includes fluid transferring step information and extraction step information.

The program configuration interface using tabs will be described as an example. When the program of the target first experimental program is configured, the program configuration interface includes a plurality of tab options corresponding to a plurality of configuration items of the target first experimental program. Any tab option is selected to display a configuration page of the corresponding configuration item. The tab options include tab options corresponding to the configuration items of the basic program information and the experimental steps. The tab option corresponding to the basic program information is selected to correspondingly display the configuration page of the basic program information, and the tab option corresponding to the experimental steps is selected to correspondingly display the configuration page of the experimental steps, so as to input relevant configuration information by the user, thereby configuring the program for the target first experimental program.

The example configures the program of the target first experimental program on the basis of the operation by the user in the program configuration interface, supports the user to flexibly input the program configuration information, has high compatibility with program configuration of a third party manufacturer, and has convenient operation.

In another example, program graphic code information is analyzed to obtain and store program configuration information of a target first experimental program.

A program graphic code refers to a graphic code for configuring the target first experimental program. Similar to a project graphic code, the program graphic code and the target first experimental program have a one-to-one relation, and the program graphic code specifically may be a two-dimensional code or a bar code. Similarly, in some embodiments, the program configuration information of the target first experimental program also includes basic program information and experimental step information, and the code scanning apparatus is also the same as described above, which are not described.

In some embodiments of the present disclosure, the user scans the program graphic code according to the code scanning apparatus, a control terminal obtains program graphic code information, analyzes the program graphic code information according to a preset decoding program to obtain the program configuration information of the target first experimental program, and stores the program configuration information. The mode does not need the user to manually fill in a large amount of information, such that the program configuration information may be efficiently and conveniently input. Moreover, the program configuration information is encapsulated in the graphic code in advance, and information contents are not disclosed to the outside, thereby being conducive to information confidentiality.

In another example, control software may support both of the above two modes. A user may perform a program information input operation in a program configuration interface, or scan a program graphic code by means of a code scanning apparatus. If control software detects the program information input operation, the control software acquires program configuration information of a target first experimental program on the basis of the operation. If the control software obtains program graphic code information, the control software analyzes the program graphic code information to obtain the program configuration information of the target first experimental program. Thus, requirements of different scenarios may be flexibly satisfied.

In some embodiments of the present disclosure, a method for configuring a test project according to the embodiments of the disclosure may further include: a sample registration interface is displayed, where the sample registration interface displays samples to be tested and candidate test projects; samples to be registered are selected from the samples to be tested on the basis of a sample selection operation in the sample registration interface; projects to be tested are determined from the candidate test projects on the basis of a project selection operation in the sample registration interface; and the projects to be tested are registered for the samples to be registered in response to a registration trigger operation.

The sample registration interface is a software interaction page for implementing a sample registration function. In a sample analysis process, after the target test project is created and configured, it is further necessary to register a sample. In some embodiments of the present disclosure, the projects to be tested are registered for the samples to be tested. In some embodiments, the sample may be registered by means of the sample registration interface.

In some embodiments of the present disclosure, the sample registration interface displays the samples to be tested, the samples to be tested correspond to target entity samples loaded in a sample analysis instrument one to one, and the target entity samples are entity samples loaded in the sample analysis instrument that need to be tested experimentally. For example, it is necessary to experimentally test N entity samples in a laboratory department. The user loads the N entity samples into the sample analysis instrument, and the N entity samples may be the target entity samples. The control software displays N samples to be tested corresponding to the N target entity samples one to one in the sample registration interface to perform relevant operations of sample registration by the user. In addition, the samples to be tested may be interface elements. For example, the sample registration interface displays sample icons, and each sample icon represents one sample to be tested. Alternatively, the sample registration interface displays a sample list, and each entry in the sample list represents one sample to be tested.

Moreover, the sample registration interface further displays the candidate test projects. The candidate test projects include all test projects created in the control software, and include the closed test projects and the open test projects described above. That is, as long as the test projects have been created in the control software, the sample registration interface displays the test projects to register the sample by the user.

The sample selection operation is an operation of selecting the samples to be registered from the samples to be tested displayed in the sample registration interface. The samples to be registered are samples to be tested that currently need sample registration. In some embodiments of the present disclosure, the sample selection operation may be a single-choice operation. That is, the user only selects one sample to be tested for separate registration at one time. Alternatively, the sample selection operation may be a multiple-choice operation. That is, the user selects a plurality of samples to be tested at one time for batch registration. In specific implementation, the sample selection operation may be a click operation for the samples to be tested, a check operation performed by correspondingly setting a check box for each sample to be tested, or a box selection operation for the samples to be tested.

The project selection operation is an operation of selecting the projects to be tested for the samples to be registered. Similarly, the project selection operation may be a single-choice operation. That is, the user only selects one project to be tested for the sample to be registered at one time for registration. Alternatively, the project selection operation may be a multiple-choice operation. That is, the user selects a plurality of projects to be tested for the sample to be registered at one time for registration. Similarly, the project selection operation may specifically use any applicable operation mode such as clicking, checking, and boxing.

In some embodiments, the control software displays the sample registration interface, and the interface lists and displays the samples to be tested and the candidate test projects. The user performs the sample selection operation in the interface, selects one or more samples to be tested as the samples to be registered, and performs the project selection operation, and selects one or more candidate test projects from the candidate test projects as the projects to be tested. Further, the control software registers the projects to be tested for the samples to be registered after the user performs the registration trigger operation. In addition, the user may repeat the above operations according to actual requirements, register the samples for the plurality of samples to be tested, and subsequently carry out corresponding experimental tests for the samples to be tested that have completed sample registration.

It should be noted that in addition to registering the projects to be tested for the samples to be tested, the sample registration may include other registration items, for example, inputting sample information for the samples to be tested. The sample information may be set according to actual requirements, and includes a sample number and a sample type, for example. Secondly, there may be various modes to trigger display of the sample registration interface. For example, the user clicks a sample registration button to trigger the display of the sample registration interface. Alternatively, the user performs a predetermined gesture operation to trigger the display of the sample registration interface. In addition, the sample registration interface may use any applicable page design solution, as long as the sample registration function may be implemented.

In some embodiments of the present disclosure, a method for configuring a test project according to the embodiments of the disclosure may further include: samples to be tested are divided into experimental batches on the basis of sample types of samples to be tested and projects to be tested in response to an experiment starting operation; and a sample analysis instrument is controlled to perform corresponding experimental operations on target entity samples corresponding to the samples to be tested in the experimental batch for each experimental batch on the basis of the sample types of the experimental batch, program mapping relations of projects to be tested of the experimental batch, and program configuration information of a second experimental program.

The experiment starting operation is an operation of triggering experimental test. In a sample analysis process, the experimental test may be started after the sample registration is completed. For example, the user clicks an experiment starting button to trigger the sample analysis instrument to start executing corresponding experimental operations.

In some embodiments, after the control software detects the experiment starting operation, the control software divides the samples to be tested into the experimental batches, and may specifically divide the samples to be tested on the basis of the sample types of the samples to be tested and the registered projects to be tested. Then, a corresponding experimental test is carried out for each experimental batch, and for each experimental batch, the sample analysis instrument is controlled to perform corresponding experimental operations on the target entity samples corresponding to the samples to be tested in the experimental batch on the basis of the sample type of the experimental batch, the program mapping relation of the projects to be tested of the experimental batch and the program configuration information of the second experimental program. In some embodiments of the present disclosure, the first experimental program corresponding to the experimental batch may be determined on the basis of the sample type of the experimental batch and the program mapping relation of the projects to be tested of the experimental batch, and the program configuration information of the first experimental program may be acquired from a database. Moreover, the program configuration information of the second experimental program of the projects to be tested of the experimental batch may be acquired, and the control sample analysis instrument may be further controlled to perform corresponding experimental operations on the target entity samples corresponding to the samples to be tested in the experimental batch on the basis of the program configuration information of the first experimental program and the program configuration information of the second experimental program.

For the specific mode of division of the experimental batches, in an embodiment, samples to be tested divided into the same experimental batch may have the same sample type and the same project to be tested.

In another embodiment, samples to be tested divided into the same experimental batch may correspond to the same first experimental program and have the same project to be tested. That is, the samples to be tested in the same experimental batch may have different sample types as long as each sample type corresponds to the same first experimental program. For example, there are five samples to be tested, the same project to be tested is registered for the five samples, but the five samples to be tested have three different sample types. Two samples to be tested S1 and S2 are a first sample type, two samples to be tested S3 and S4 are a second sample type, and a sample to be tested S5 is a third sample type. In a program mapping relation, the first sample type and the second sample type correspond to the same first experimental program, and the third sample type corresponds to another different first experimental program. Thus, when the samples to be tested are divided into the experimental batches, the samples to be tested S1-S4 may be divided into the same experimental batch, and the sample to be tested S5 is divided into another experimental batch.

To this end, the inventor finds that in the prior art, it is necessary to create and configure a plurality of test projects for the same test project, and a control terminal controls a sample analysis instrument to perform experimental test. When experimental test is carried out in batches, only one test project is arranged in one experimental batch. For a plurality of samples to be tested, even if the same test project is carried out, if the sample types are different, the samples to be tested may be registered as different test projects accordingly, and are further divided into different experimental batches for test. As shown in the above example, a COVID nucleic acid test project is carried out on ten samples to be tested. Three throat swabs, three serum and four feces are registered as three COVID nucleic acid test projects P1, P2 and P3 respectively, and are further divided into three experimental batches. Thus, the number of the experimental batches is increased, resulting in extension of experimental test time and waste of experimental reagents and consumables. However, in the embodiments of the disclosure, the first experimental programs corresponding to different sample types are configured for one test project, and the samples to be tested in the same experimental batch correspond to the same first experimental program and have the same project to be tested. Thus, more samples to be tested may be distributed into the same experimental batch for experimental test, thereby reducing the experimental batches. Thus, experimental time is saved, and waste of experimental resources is reduced.

In addition, it should be noted that in a sample analysis process, the control software integrates a number of information to control the sample analysis instrument to perform experimental operation. Only parts relevant to the first experimental program and the second experimental program of the project to be tested are only described above. Other parts such as quality control and reagent are not the focus of the embodiments of the disclosure and may be implemented in any applicable mode in the art, which are not described herein.

For ease of a clear and intuitive understanding, the contents mainly involved in the embodiments of the disclosure will be described below in combination with a specific application example:
in some embodiments applied to the field of nucleic acid test, COVID nucleic acid test is carried out on ten samples. Three throat swabs, three serum and four feces are included. The three sample types correspond to nucleic acid extraction programs TQ1, TQ2 and TQ3 respectively, and a COVID nucleic acid test project corresponds to a nucleic acid amplification program KZ.

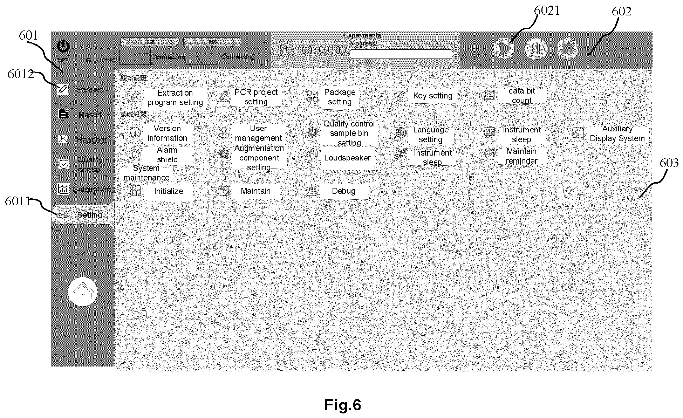

In some embodiments of the present disclosure, an application environment includes a control terminal and a PCR integrated machine. Control software is installed in the control terminal. The control software is provided with main function components of a sample component, a result component, a reagent component, a quality control component, a calibration component, and a setting component. An interface of the control software is as shown in Fig. 6. A left side of the interface is provided with a navigation area 601, a top of the interface is provided with an experiment control area 602, and a middle of the interface is provided with a function area 603. The navigation area 601 is provided with function buttons of a sample button (6012), a result button, a reagent button, a quality control button, a calibration button, a setting button (6011) and a home page button, and any function button is clicked to display a corresponding function interface in the function area 603. The experiment control area 602 is provided with function buttons of an experiment starting button (6021), a sample addition pause button and a shutdown button, and any function button is clicked to control the PCR integrated machine to enter a corresponding working state.

COVID nucleic acid test is carried out on the above ten samples, and mainly involves relevant contents of four aspects of program configuration of nucleic acid extraction programs, configuration of the COVID nucleic acid test project, sample registration and experimental test control, which are introduced respectively as follows:

### 1. Program configuration of nucleic acid extraction programs

It is necessary to configure programs for the three nucleic acid extraction programs TQ1, TQ2 and TQ3 at first respectively, so as to obtain and store program configuration information of the three nucleic acid extraction programs.

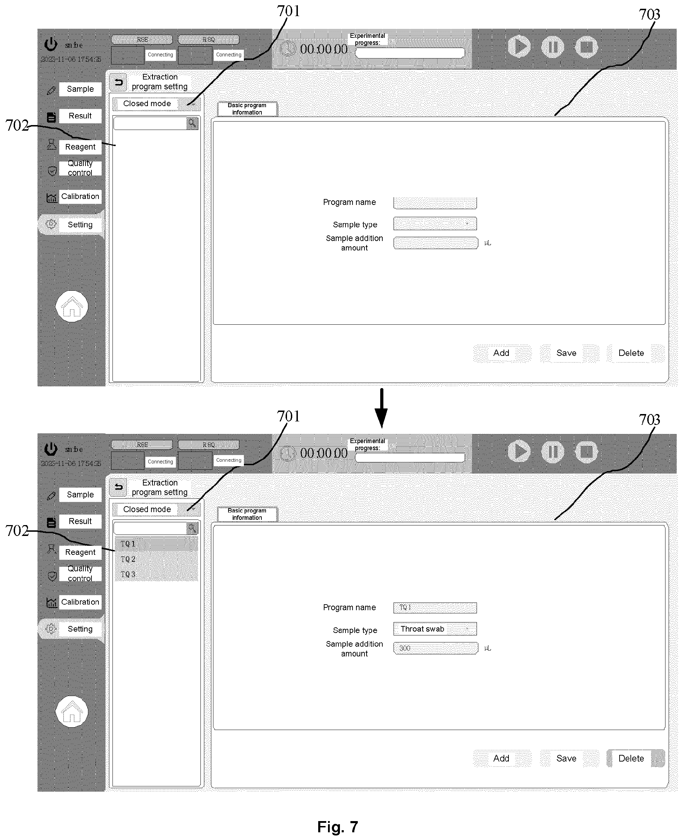

A user clicks the setting button 6011 in the navigation area 601, the function area 603 displays a software setting interface, and then, the user clicks an extraction program setting button in the interface. As shown in an upper figure of Fig. 7, control software displays a program configuration interface. The program configuration interface includes a mode drop-down box 701, a program list area 702, and a program editing area 703. The control software supports two information input modes of a closed mode and an open mode. When a closed option is selected in the mode drop-down box 701, the program list area 702 centrally displays all nucleic acid extraction programs (hereinafter referred to as closed nucleic acid extraction programs) created by means of program two-dimensional codes in a form of a list. Moreover, a specific nucleic acid extraction program may be selected in the program list area 702 to further view relevant contents in the program editing area 703. When the open option is selected in the mode drop-down box 701, the program list area 702 centrally displays all the nucleic acid extraction programs (hereinafter referred to as open nucleic acid extraction programs) created on the basis of an operation by the user in the program configuration interface in a form of a list. Moreover, a specific nucleic acid extraction program may be selected in the program list area 702 to further view and edit relevant contents in the program editing area 703.

The processes of creating and configuring the three nucleic acid extraction programs TQ1, TQ2 and TQ3 in the control software by means of the closed mode and the open mode will be introduced respectively below:

### 1) Closed mode

A user scans a program two-dimensional code corresponding to the nucleic acid extraction program TQ1 by means of a code scanning gun, and the control software analyzes program two-dimensional code information to obtain program configuration information of the nucleic acid extraction program TQ1. The program configuration information specifically includes relevant configuration information of three configuration items of basic program information of the nucleic acid extraction program TQ1, a fluid transferring step and an extraction step. The basic program information includes a program name, a sample type and a sample addition amount, the fluid transferring step includes a reagent component and a sample addition amount, and the extraction step includes step distribution information and parameter setting information (for example, parameters such as time, a rotation speed and a temperature of pyrolysis, magnetic bead attraction, binding, washing I and washing II) of each step. Further, the control software associatively stores a program identifier of the nucleic acid extraction program TQ1 and the program configuration information to create and configure the nucleic acid extraction program TQ1. Subsequently, the above steps are repeated to create and configure the nucleic acid extraction programs TQ2 and TQ3.

Further, as shown in a lower figure of Fig. 7, when the mode drop-down box 701 in the program configuration interface corresponds to the closed option, the program list area 702 of the interface displays the nucleic acid extraction programs TQ1, TQ2 and TQ3 in a form of a list. Moreover, the user selects the closed nucleic acid extraction program TQ1 in the program list area 702, and the program editing area 703 displays a configuration page of the basic program information, but does not display configuration pages of the fluid transferring step and the extraction step. The program name, the configuration page of the basic program information specifically displays the sample type and the sample addition amount of the nucleic acid extraction program TQ1, and the public configuration information is only viewed and not edited by the user. It should be noted that the program list area 702 displays all the closed nucleic acid extraction programs created, and if other closed nucleic acid extraction programs are created in addition to TQ 1 to TQ3, the program list area displays other closed nucleic acid extraction programs together. Moreover, clicking of any nucleic acid extraction program in the program list area 702 by the user is similar to clicking of TQ1, which is not described herein.

### 2) Open mode

Public configuration items of the open extraction program are preset in the control software to include three configuration items of basic program information, a fluid transferring step and an extraction step, and public configuration information of each public configuration item is preset according to requirements. For example, the public configuration information of the basic program information includes a program name, a sample type and a sample addition amount, and the public configuration information of the fluid transferring step includes a reagent component and a sample addition amount, all of which are set as editable contents.

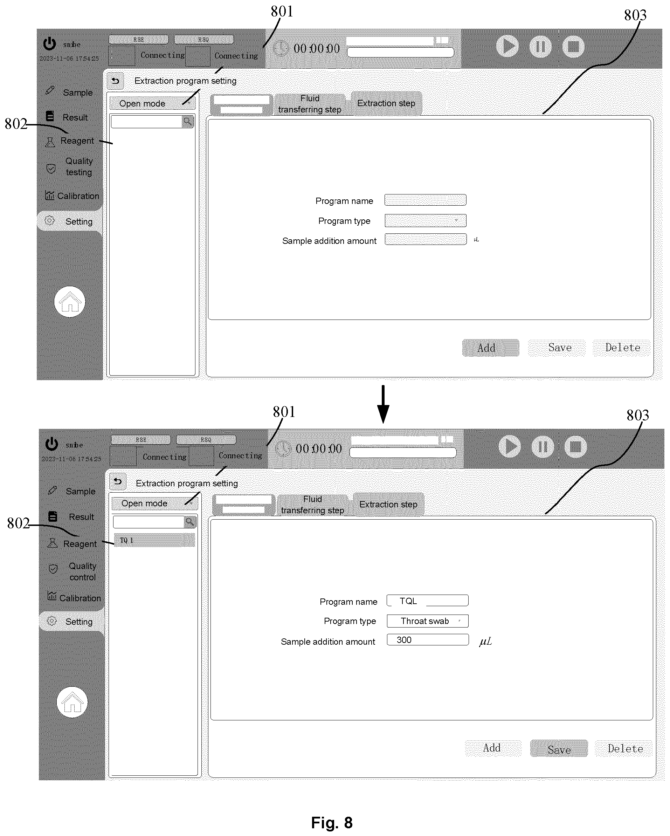

On this basis, as shown in an upper figure of Fig. 8, control software displays a program configuration interface, a user selects an open option in a mode drop-down box 801 in the interface, and a program list area 802 of the interface centrally displays all open nucleic acid extraction programs in a form of a list (the upper figure of Fig. 8 corresponds to a case in which no open nucleic acid extraction program has been created in the control software, and the program list area 802 is blank). A program editing area 803 displays three tab options corresponding to the three public configuration items of the basic program information, the fluid transferring step and the extraction step, the three tab options correspond to configuration pages of the three public configuration items respectively, and the program editing area 803 displays the configuration page of the public configuration item corresponding to the selected tab option. In some embodiments of the present disclosure, the control software selects the tab option corresponding to the basic program information by default. Thus, the user selects the open option in the mode drop-down box 801, and the program editing area 803 directly displays the configuration page of the basic program information. Subsequently, the user selects the tab option corresponding to the fluid transferring step instead, and the program editing area 803 is switched to display the configuration page of the fluid transferring step.

The configuration page of the basic program information is provided with an add button. The user clicks the add button, the control software automatically inputs the program name (for example, the string code of "20231213100907" is automatically filled, but the user is allowed to modify the program name, and as shown in a lower figure of Fig. 8, the user modifies the program name to TQ1) of the nucleic acid extraction program to be added in a program name field of the configuration page. Further, the user manually inputs the sample type and the sample addition amount of the nucleic acid extraction program TQ1 in the configuration page, and then clicks a save button in the configuration page. The control software associatively stores a program identifier of the nucleic acid extraction program TQ1 and the program configuration information input above, so as to create the nucleic acid extraction program TQ1 and configure the configuration item of the basic program information for the nucleic acid extraction program. Moreover, after the user clicks the save button, as shown in the lower figure of Fig. 8, the nucleic acid extraction program TQ1 is added and displayed in the program list area 801 and is automatically selected.

Further, the user selects the tab option corresponding to the fluid transferring step in the program editing area 803, and the control software displays the configuration page of the fluid transferring step. The user manually inputs the relevant configuration information of the fluid transferring step of the nucleic acid extraction program TQ1 in the configuration page, and the control software associatively stores the information input by the user and the program identifier of the nucleic acid extraction program TQ1, so as to configure the configuration item of the fluid transferring step for the nucleic acid extraction program TQ1. Moreover, the user clicks the tab option corresponding to the configuration item of the extraction step, and the control software displays the configuration page of the extraction step. The user manually inputs the configuration information relevant to the extraction step in the configuration page, and the control software associatively stores the information input by the user and the program identifier of the nucleic acid extraction program TQ1, so as to configure the configuration item of the extraction step for the nucleic acid extraction program TQ1. So far, the control software completes creation and configuration of the open nucleic acid extraction program of TQ1.

Then, the above steps are repeated to create and configure the nucleic acid extraction programs TQ2 and TQ3 (not shown).

### 2. Configuration of nucleic acid test project

After the nucleic acid extraction programs TQ1, TQ2 and TQ3 are created and configured, the COVID nucleic acid test project is created and configured.

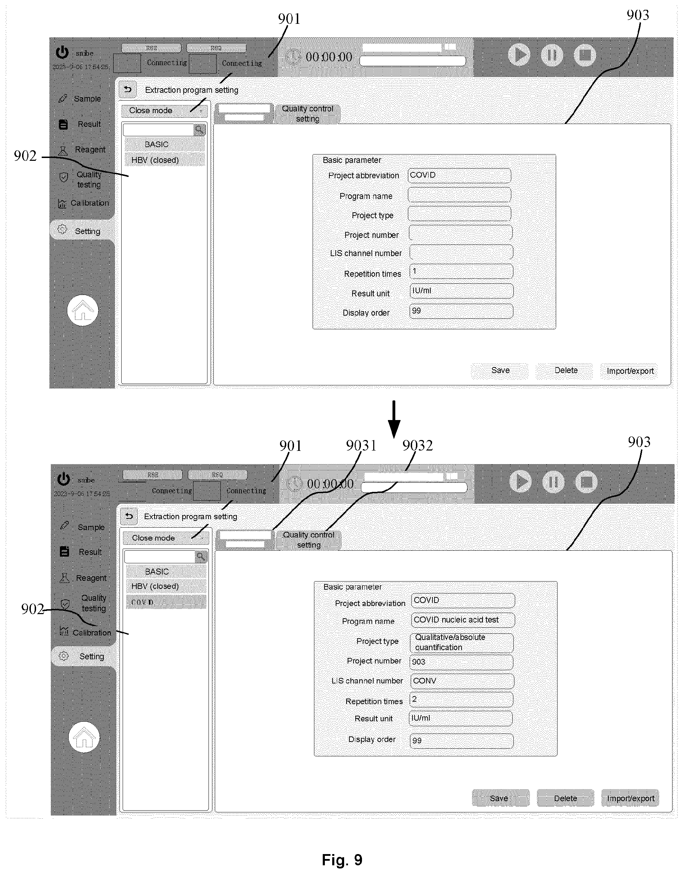

The user clicks a PCR project setting button in the software setting interface shown in Fig. 6. As shown in an upper figure of Fig. 9, control software displays a project configuration interface. The project configuration interface includes a mode drop-down box 901, a project list area 902, and a project editing area 903. Similarly, the control software supports two information input modes of the closed mode and the open mode. When the closed option is selected in the mode drop-down box 901, the project list area 902 centrally displays all the nucleic acid test projects (hereinafter referred to as closed nucleic acid test projects) created by means of project two-dimensional codes in a form of a list, and a specific closed nucleic acid test project may be selected in the project list area 902 to further view and edit relevant contents in the project editing area 903. When the open option is selected in the mode drop-down box 901, the project list area 902 centrally displays all the nucleic acid test projects (hereinafter referred to as open nucleic acid test projects) created on the basis of the operation by the user in the project configuration interface in a form of a list, and a specific open nucleic acid test project may be selected from the project list area 902 to further view and edit relevant contents in the project editing area 903 (It should be noted that as shown in the upper figure of Fig. 9, it is assumed that the control software only creates two closed nucleic acid test projects of "HPV (closed)" and "hepatitis B virus (HBV) (closed)" before the COVID nucleic acid test project is created, and thus the project list area 902 only displays the two closed nucleic acid test projects).

The processes of creating and configuring the COVID nucleic acid test projects in the control software by means of the closed mode and the open mode will be introduced respectively below:

### 1) Closed mode

The user scans the two-dimensional code corresponding to the COVID nucleic acid test project by means of the code scanning gun, and the control software analyzes project two-dimensional code information to obtain project configuration information of the COVID nucleic acid test project. The project configuration information specifically includes relevant configuration information of seven configuration items of basic project information of the COVID nucleic acid test project, a reagent component, a test target, result determination, the nucleic acid extraction program, the nucleic acid amplification program and a quality control setting. Further, the control software associatively stores the project identifier of the COVID nucleic acid test project and the project configuration information. So far, the COVID nucleic acid test project is created and configured.

With regard to the basic project information, the project two-dimensional code is analyzed to specifically obtain a project abbreviation of the COVID nucleic acid test project, a project name, a project type, a project number, an LIS channel number, repetition times, a result unit, a display order, a nucleic acid sample addition amount and an exogenous internal standard. With regard to the nucleic acid extraction programs, a program mapping relation of the COVID nucleic acid test project is obtained. That is, the three sample types of the throat swab, the serum and the feces and the three nucleic acid extraction procedures TQ1, TQ2 and TQ3 have one-to-one mapping relations. With regard to the nucleic acid amplification program, program configuration information of the nucleic acid amplification program KZ is obtained, and may specifically include program segment distribution information and specific program segment setting information introduced above. The four configuration items of the reagent component, the test target, the result determination and the quality control setting are not key contents of the embodiments of the disclosure, and relevant configuration information may satisfy conventional requirements in the art, which is not described herein.

Further, the mode drop-down box 901 in the project configuration interface corresponds to the closed option. As shown in a lower figure of Fig. 9, the project list area 902 of the interface centrally displays all the closed nucleic acid test projects that have been created in a form of a list. The closed nucleic acid test projects include HPV (closed), HBV (closed) and the COVID nucleic acid test project.

Moreover, the user selects the COVID nucleic acid test project in the project list area 902, the project editing area 903 displays tab options 9031 and 9032 corresponding to the two configuration items of the basic project information and the quality control setting, the two tab options correspond to a configuration page of basic project information and a configuration page of the quality control setting respectively, and the project editing area 903 displays the configuration page of the public configuration item corresponding to the selected tab option. In some embodiments of the present disclosure, the control software selects the tab option 9031 corresponding to the basic project information by default. Thus, the project editing area 903 displays the configuration page of the basic project information of the COVID nucleic acid test project by default. Subsequently, the user selects the tab option 9032 corresponding to the quality control setting instead, and the project editing area 903 is switched to display the configuration page of the quality control setting of the COVID nucleic acid test project. Moreover, in some embodiments of the present disclosure, part of the information displayed in the configuration page, such as the project abbreviation, the project name, the project type and the project number in the basic project, is only viewed but not edited by the user, and another part of the information, such as the LIS channel number, the repetition times, the result unit and the display order in the basic project information and a quality control type required for each batch of experiments in the quality control setting, is viewed and edited by the user.

### 2) Open mode

The public configuration items of the open nucleic acid test project are preset in the control software to include seven configuration items of basic project information, a reagent component, a test target, result determination, the nucleic acid extraction program, the nucleic acid amplification program and a quality control setting. The open configuration information of the basic project information includes a project abbreviation, a project name, a project type, a project number, an LIS channel number, repetition times, a result unit, a display order, a nucleic acid sample addition amount and an exogenous internal standard. The public configuration information of the nucleic acid extraction program includes a program mapping relation, i.e. mapping relations between sample types and the nucleic acid extraction programs. The public configuration information of the nucleic acid amplification program includes program segment distribution information and specific program segment setting information. The four configuration items of the reagent component, the test target, the result determination, and the quality control setting are not key contents of the embodiments of the disclosure, and the public configuration information of the four configuration items only may satisfy conventional requirements in the art, are not described herein, and are set as editable contents.

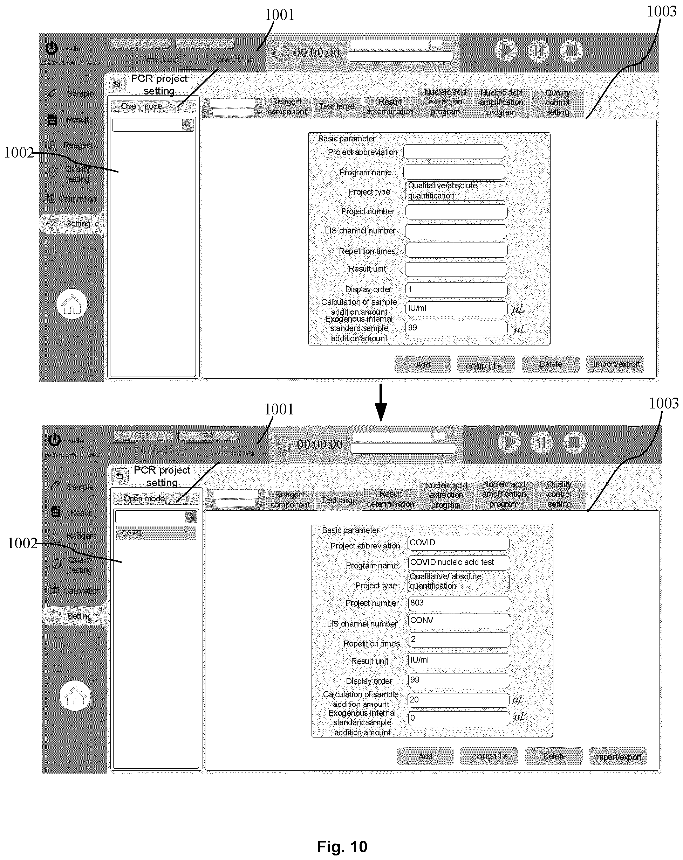

On this basis, as shown in an upper figure of Fig. 10, control software displays a project configuration interface, a user selects an open option in a mode drop-down box 1001 in the interface, and a project list area 1002 centrally displays all the created open nucleic acid extraction projects in a form of a list (the upper figure of Fig. 10 corresponds to a case in which no open nucleic acid test project has been created in control software, and the project list area 1002 is blank). A project editing area 1003 displays seven tab options corresponding to the seven public configuration items, and the seven tab options correspond to configuration pages of the seven public configuration items respectively. The project editing area 1003 displays the configuration page of the public configuration item corresponding to the selected tab option. In some embodiments of the present disclosure, the control software selects the tab option corresponding to the basic project information by default. Thus, the user selects the open option in the mode drop-down box 1001, and the project editing area 1003 directly displays the configuration page of the basic project information. Subsequently, the user selects the tab option corresponding to the nucleic acid extraction program instead, and the project editing area 1003 is switched to display the configuration page of the nucleic acid extraction program.

The configuration page of the basic project information is provided with an add button. The user clicks the add button to further manually input the basic project information of the COVID nucleic acid test project in the configuration page. The basic project information specifically includes a project abbreviation, a project name, and a project type. Further, the user clicks a save button, and the control software associatively stores the input basic project information and a project identifier of the COVID nucleic acid test project, so as to create the COVID nucleic acid test project and configure the configuration item of the basic project information for the COVID nucleic acid test project. Moreover, after the user clicks the save button, as shown in a lower figure of Fig. 10, the COVID nucleic acid test project is added and displayed in the project list area 1002 and is automatically selected.

Further, the user selects the tab option corresponding to the nucleic acid extraction program in the project editing area 1003, the control software displays the configuration page of the nucleic acid extraction program of the COVID nucleic acid test project, and the user performs relevant operations in the interface to input the sample type and the nucleic acid extraction program for the COVID nucleic acid test project.

Reference may be made to the example corresponding to Fig. 3 above for details. The user may click the add button in the configuration page, and the control software displays an extraction program setting pop-up box. The pop-up box includes a sample type drop-down box and a nucleic acid extraction program drop-down box. The user selects a throat swab sample type in the sample type drop-down box, and the control software obtains a corresponding type identifier, and determines the nucleic acid extraction program associated with the throat swab sample type on the basis of the type identifier and the program configuration information of the plurality of nucleic acid extraction programs stored in advance. It is assumed that the program configuration information of only the three nucleic acid extraction programs TQ1, TQ2, and TQ3 is stored in advance, such that the nucleic acid extraction program TQ1 is determined as the nucleic acid extraction program associated with the throat swab sample type, and the nucleic acid extraction program drop-down box only displays the nucleic acid extraction program TQ1. The user selects the nucleic acid extraction program TQ1, and the control software obtains the program identifier of the nucleic acid extraction program TQ1. The user clicks a save button in the pop-up box, and the control software associatively stores the type identifier of the throat swab sample type and the program identifier of the nucleic acid extraction program TQ1 to obtain the mapping relations between the throat swab sample type and the nucleic acid extraction program. That is, the throat swab sample type is input for the COVID nucleic acid test project, and the corresponding nucleic acid extraction program TQ1 is determined for the throat swab sample type. Subsequently, the above operations are repeated, the two sample types of the serum and the feces continue being input for the COVID nucleic acid test project, and the corresponding nucleic acid extraction programs TQ2 and TQ3 are determined for the COVID nucleic acid test project. So far, the control software obtains the one-to-one mapping relations between the three sample types and the three nucleic acid extraction programs, i.e. the program mapping relation of the COVID nucleic acid test project.

Moreover, the user selects the tab option corresponding to the nucleic acid amplification program in the project editing area 1003, and the control software displays the configuration page of the nucleic acid amplification program. The user manually inputs the program configuration information of the nucleic acid amplification program in the configuration page, and clicks the save button, and the control software associatively stores the basic program information input by the user and the project identifier of the COVID nucleic acid test project to configure the nucleic acid amplification program for the project.

In addition, the user selects the tab options corresponding to the reagent component, the test target, the result determination and the quality control settings separately, and the control software displays the corresponding configuration pages separately. The user manually inputs the corresponding configuration information of the COVID nucleic acid test project in the configuration pages, and the control software configures corresponding configuration items for the COVID nucleic acid test project. However, the configuration items are not key contents of the embodiments of the disclosure and may be implemented in any applicable mode, which are not described herein.

So far, the control software creates and configures the COVID nucleic acid test project.

### 3. Sample registration

After the COVID nucleic acid test project is created and configured, sample registration is carried out on the above ten samples to input sample information for the samples and register projects to be tested.

In some embodiments of the present disclosure, the user places the ten samples into a sample bin of the PCR integrated machine, and further, the user clicks a sample button 6012 in the navigation area 601 shown in Fig. 6, and the control software displays the sample registration interface (not shown). The sample registration interface displays ten samples to be tested corresponding to the ten samples one to one, and the user inputs sample information for the ten samples to be tested in the sample registration interface respectively, and inputs the sample types, for example. In some embodiments of the present disclosure, the sample type input for three samples to be tested is the throat swab, the sample type input for the other three samples is the serum, and the sample type input for the remaining four samples is the feces. Further, the user checks the ten samples to be tested, and clicks a project registration button in the sample registration interface, the control software displays a project registration pop-up box, and all the test projects created in the control software are displayed in the pop-up box. The test projects include the COVID nucleic acid test project. The user selects the COVID nucleic acid test project and clicks an ok button, and the control software binds sample identifiers of the ten samples to be tested with a project identifier of the COVID nucleic acid test project. That is, the COVID nucleic acid test project is registered for the ten samples to be tested.

### 4. Experimental test

After the sample registration is completed, the user may click the experiment starting button 6021 in the experiment control area 602 shown in Fig. 6 subsequently, and the control software controls the PCR integrated machine to execute relevant experimental operations.

In some embodiments of the present disclosure, the control software divides the samples to be tested into experimental batches, the samples to be tested divided into the same experimental batch have the same sample type and the same project to be tested, and the COVID nucleic acid test project is registered for the ten samples to be tested. Thus, the three sample types are correspondingly divided into three experimental batches. Further, the control software controls the PCR integrated machine to perform corresponding experimental operations on each experimental batch. The experimental batch in which the three throat swab samples to be tested are located is taken as an example, the control software controls the PCR integrated machine to perform operations of extraction plate configuration, amplification plate configuration, sample addition, nucleic acid extraction, nucleic acid transfer and nucleic acid amplification in corresponding experimental operation modes on the basis of the type identifier of the throat swab sample type and the project configuration information of the COVID nucleic acid test project. Thus, the PCR integrated machine obtains experimental test data of the three samples to be tested and sends the experimental test data to the control software.

It should be noted that for the nucleic acid extraction, the control software searches for the program identifier of the nucleic acid extraction program TQ1 from the program mapping relation of the COVID nucleic acid test project according to the type identifier of the throat swab sample type and the project identifier of the COVID nucleic acid test project, and searches for the program configuration information of the nucleic acid extraction program TQ 1 on the basis of the program identifier. Subsequently, the control software controls the PCR integrated machine to perform nucleic acid extraction processing according to an experimental operation mode indicated in the program configuration information. For the nucleic acid amplification, the control software searches for the program configuration information of the nucleic acid amplification program of the COVID nucleic acid test project according to the project identifier of the COVID nucleic acid test project, and subsequently controls the PCR integrated machine to perform nucleic acid amplification processing according to an experimental operation mode indicated in the program configuration information. In addition, as described above, the control software controls the PCR integrated machine to complete all the experimental operations for one experimental batch, which involves a number of contents, such as quality control processing and calibration processing, is not the focus of the embodiments of the disclosure, may be implemented in any applicable mode in the art, and is not described herein.

Subsequently, the control software controls the PCR integrated machine to perform corresponding experimental operations in a similar mode for the experimental batches in which the three serum samples to be tested and the four feces samples to be tested are located respectively, so as to obtain experimental test data of the seven samples to be tested and send the experimental test data to the control software, which is not described for details. So far, COVID nucleic acid test of the ten samples is completed.

It should be understood, where appropriate, that although various steps in the flow diagrams involved in the above examples are sequentially shown as indicated by arrows, the steps are not necessarily performed sequentially in the order indicated by the arrows. Unless explicitly stated herein, there is no strict order limitation for execution of the steps, and the steps may be executed in other orders. Moreover, at least part of the steps in each flow diagram may include a plurality of sub-steps or a plurality of stages, which are not necessarily completed at the same time, but can be executed at different times. The order of execution of these sub-steps or stages is not necessarily sequential, but may be rotated or alternated with at least a portion of the sub steps or stages of other steps or steps.

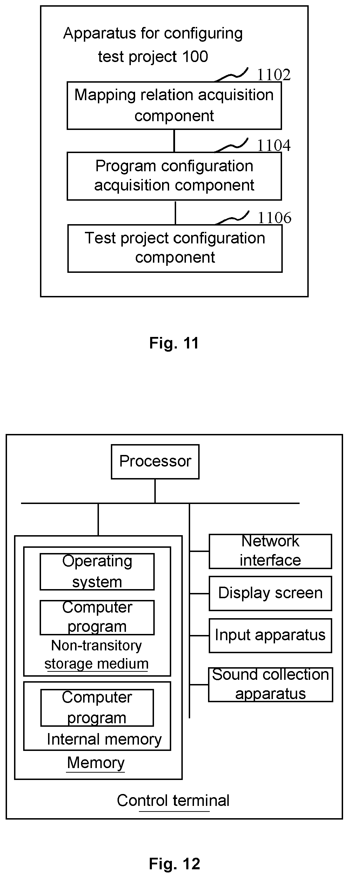

In some embodiments of the present disclosure, as shown in Fig. 11, an apparatus 1100 for configuring a test project is further provided. The apparatus may include the following components 1102-1106:
a mapping relation acquisition component 1102, configured to acquire a program mapping relation of a target test project, where the program mapping relation includes mapping relations between a plurality of target sample types and at least one target first experimental program;
a program configuration acquisition component 1104, configured to acquire program configuration information of a target second experimental program, where the target second experimental program uniquely corresponds to a project type of the target test project; and
a test project configuration component 1106, configured to configure the target test project on the basis of the program mapping relation and the program configuration information of the target second experimental program.

The apparatus for configuring a test project acquires the mapping relations between the plurality of sample types corresponding to the target test project and the at least one first experimental program, acquires the program configuration information of the second experimental program uniquely corresponding to the project type of the target test project, and configures the target test project on the basis of the mapping relations and the program configuration information. Thus, the first experimental programs corresponding to different sample types can be configured for one test project. For a case in which one test project has the plurality of sample types, it is only necessary to create one test project and unnecessary to create one test project for each sample type, thereby simplifying workload of project configuration and later modification, and improving convenience and efficiency of the project configuration. Moreover, only one test project can be displayed for the same test project in the sample registration interface, thereby effectively avoiding confusion when the user registers a sample, and improving registration efficiency and accuracy.

In some embodiments of the present disclosure, a mapping relation acquisition component 1102 includes a sample type determination component, a first experimental program determination component, and a mapping relation obtaining component. The sample type determination component is configured to determine a plurality of target sample types on the basis of a sample type input operation by a user in a project configuration interface; the first experimental program determination component is configured to determine a first experimental program associated with each target sample type on the basis of program configuration information of first experimental programs stored in advance, and determine the target first experimental program corresponding to the target sample type from the associated first experimental program on the basis of a program selection operation by the user in the project configuration interface; and the mapping relation obtaining component is configured to obtain the program mapping relation of the target test project on the basis of the plurality of target sample types determined and the target first experimental programs corresponding to the target sample types.

In some embodiments of the present disclosure, an apparatus 1100 for configuring a test project includes a basic project information acquisition component and a first test project creation component. The basic project information acquisition component is configured to acquire basic project information of a target test project on the basis of a basic information input operation by a user in a project configuration interface; and the first test project creation component is configured to create the target test project on the basis of the basic project information in response to a project creation operation by the user. On this basis, a test project configuration component 1106 includes a test project update component, configured to update the target test project on the basis of a program mapping relation and program configuration information of a target second experimental program.

In some embodiments of the present disclosure, a mapping relation acquisition component 1102 includes a first analysis component, configured to analyze project graphic code information to obtain the program mapping relation of the target test project, where the project graphic code information is obtained by means of scanning by a code scanning apparatus.

In some embodiments of the present disclosure, an apparatus 1100 for configuring a test project includes a second analysis component, configured to analyze a project graphic code to obtain basic project information of the target test project. On this basis, a program configuration acquisition component 1104 includes a third analysis component, configured to analyze the project graphic code to obtain program configuration information of a target second experimental program. Moreover, a test project configuration component 1106 includes a second test project creation component, configured to create and configure the target test project on the basis of the basic project information, a program mapping relation and the program configuration information of the target second experimental program.

In some embodiments of the present disclosure, an apparatus 1100 for configuring a test project includes: a configuration interface display component, configured to display a project configuration interface, where the project configuration interface includes a mode selection control, a project list area and a project editing area, where the mode selection control includes an open option and a closed option; an open project display component, configured to display open test projects in the project list area when the mode selection control corresponds to the open option, where the open test projects include test projects created on the basis of an operation by a user in the project configuration interface; an open configuration page display component, configured to display a configuration page corresponding to a selected open test project in the project editing area on the basis of a selection operation for the displayed open test projects, where the configuration page corresponding to the open test project includes a configuration page of a first experimental program and a configuration page of a second experimental program; a closed project display component, configured to display closed test projects in the project list area when the mode selection control corresponds to the closed option, where the closed test projects include test projects created by means of project graphic codes; and a closed configuration page display component, configured to display a configuration page corresponding to a selected closed test project in the project editing area on the basis of a selection operation for the displayed closed test projects, where the configuration page corresponding to the closed test project does not include a configuration page of the first experimental program and a configuration page of the second experimental program.

In some embodiments of the present disclosure, an apparatus 1100 for configuring a test project includes a first experimental program configuration component, specifically configured to execute at least one of the following:
item 1: obtaining and storing program configuration information of a target first experimental program on the basis of a program information input operation by a user in a program configuration interface, where the program configuration information includes basic program information of a target first experimental program and experimental step information; and item 2: analyzing program graphic code information to obtain and store program configuration information of a target first experimental program, where the program graphic code information is obtained by means of scanning by a code scanning apparatus; and the program configuration information includes basic program information of a target first experimental program and experimental step information.

In some embodiments of the present disclosure, an apparatus 1100 for configuring a test project includes a registration interface display component, a to-be-registered sample determination component, a to-be-tested project determination component, and a registration component. The registration interface display component is configured to display a sample registration interface, where the sample registration interface displays samples to be tested and candidate test projects, and the samples to be tested correspond to target entity samples loaded in a sample analysis instrument one to one; the to-be-registered sample determination component is configured to determine samples to be registered from the samples to be tested on the basis of a sample selection operation in the sample registration interface; the to-be-tested project determination component is configured to determine projects to be tested from the candidate test projects on the basis of a project selection operation in the sample registration interface; and the registration component is configured to register the projects to be tested for the samples to be registered in response to a registration trigger operation.

In some embodiments of the present disclosure, an apparatus 1100 for configuring a test project includes an experimental batch division component and an experimental control component. The experimental batch division component is configured to divide samples to be tested into experimental batches on the basis of sample types of the samples to be tested and projects to be tested in response to an experiment starting operation, where the samples to be tested in the same experimental batch have the same sample type and the same project to be tested, and alternatively, the samples to be tested in the same experimental batch correspond to the same first experimental program and have the same project to be tested; and the experimental control component is configured to control a sample analysis instrument to perform corresponding experimental operations on target entity samples corresponding to the samples to be tested in the experimental batch for each experimental batch on the basis of the sample types of the experimental batch, program mapping relations of the projects to be tested of the experimental batch, and program configuration information of a second experimental program.

It should be noted that for the specific limitation of the apparatus 1100 for configuring a test project, reference may be made to the limitation of the method for configuring a test project above, which is not described herein. Each component in the apparatus 1100 for configuring a test project can be implemented entirely or partially by software, hardware, or a combination of the software and the hardware. Each component may be embedded into or independent of a processor in a computer device in a form of hardware, or may be stored in a memory in the computer device in a form of software, such that the processor invokes and executes an operation corresponding to each component.

In some embodiments of the present disclosure, a control terminal is provided. The control terminal includes a memory and a processor. The memory stores a computer program, and the processor implements the steps of the method for configuring a test project according to any example of the embodiments of the disclosure when executing the computer program.

In some embodiments of the present disclosure, an internal structure of the control terminal may be as shown in Fig. 12, and includes a processor, a memory, a network interface, a display screen, an input apparatus and a sound collection apparatus connected by means of a system bus. The processor is configured to provide computing and control capabilities. The memory includes a non-transitory storage medium and an internal memory. The non-transitory storage medium of the memory stores an operating system and a computer program, and the computer program may cause the processor to implement the method for configuring a test project according to any example of the embodiments of the disclosure when executed by the processor; and the internal memory provides an environment for running of the operating system and the computer program in the nonvolatile storage medium. The network interface is configured to communicate with an external terminal by means of a network connection. The display screen may be a liquid crystal display screen or an electronic ink display screen. The input apparatus may be a touch layer covering the display screen, or a key, trackball, or touch pad arranged on a shell of a computer device, or an external keyboard, touch pad, or mouse. Those skilled in the art can understand that the structure shown in Fig. 12 is only a block diagram of a part of a structure relevant to the solution of the embodiments of the disclosure, and does not constitute a limitation on the control terminal to which the solution of the embodiments of the disclosure is applied. The specific control terminal may include more or less components than those shown in Fig. 12, or combine some components, or have different component configurations.

In some embodiments of the present disclosure, an apparatus for configuring a test project according to the embodiments of the disclosure may be implemented in a form of a computer program, and the computer program may run on the control terminal as shown in Fig. 12. The memory of the control terminal may store various program modules constituting the apparatus, for example, a mapping relation acquisition component 1102 and a program configuration acquisition component 1104 shown in Fig. 11. The computer program constituted by each program module enables the processor to execute the steps in the method for configuring a test project described in each example of the embodiments of the disclosure. For example, the control terminal shown in Fig. 12 may execute S202 by means of the mapping relation acquisition component 1102 in an apparatus 1100 for configuring a test project shown in Fig. 11, and execute S204 by means of the program configuration acquisition component 1104.

Those skilled in the art can understand that all or part of the processes in the method of the above example can be completed by instructing relevant hardware by means of the computer program. The program can be stored in a nonvolatile non-transitory computer-readable storage medium, and the program may include the flow of the examples of the above methods when executed. Any reference to the memory, storage, a database, or other media used in some embodimentss according to the embodiments of the disclosure may include a nonvolatile memory and/or a volatile memory. The nonvolatile memory may include a read-only memory (ROM), a programmable ROM (PROM), an electrically programmable ROM (EPROM), an electrically erasable programmable ROM (EEPROM), or a flash memory. The volatile memory may include a random access memory (RAM) or an external cache memory. As an illustration rather than a limitation, the RAM is available in various forms, such as a static RAM (SRAM), a dynamic RAM (DRAM), a synchronous DRAM (SDRAM), a double data rate SDRAM (DDRSDRAM), an enhanced SDRAM (ESDRAM), a synchlink DRAM (SLDRAM), a rambus direct RAM (RDRAM), a direct rambus dynamic RAM (DRDRAM), and a rambus dynamic RAM (RDRAM).

Accordingly, in some embodiments of the present disclosure, a non-transitory computer-readable storage medium is provided. The non-transitory computer-readable storage medium stores a computer program, and the computer program causes a processor to execute the steps of the method for configuring a test project according to any example of the embodiments of the disclosure when executed by the processor.

In some embodiments of the present disclosure, a sample analysis system is provided. The sample analysis system includes a sample analysis instrument and the control terminal described above. The control terminal is configured to obtain and store a program mapping relation of a target test project and program configuration information of a target second experimental program, where the program mapping relation includes mapping relations between a plurality of target sample types and at least one target first experimental program, and the target second experimental program uniquely corresponds to a project type of the target test project; the control terminal is further configured to divide samples to be tested into experimental batches on the basis of sample types of the samples to be tested and projects to be tested, where the samples to be tested in the same experimental batch have the same sample type and the same project to be tested, and alternatively, the samples to be tested in the same experimental batch correspond to the same first experimental program and have the same project to be tested; and send an experimental control instruction to the sample analysis instrument for each experimental batch on the basis of the sample types of the experimental batch, program mapping relations of the projects to be tested of the experimental batch, and program configuration information of a second experimental program; and the sample analysis instrument is configured to perform corresponding experimental operations on entity samples corresponding to the samples to be tested in each experimental batch on the basis of the experimental control instruction.

Various technical features of the above examples can be arbitrarily combined. To simplify description, all possible combinations of the various features of the above examples are not described. However, if only the combinations of these technical features do not conflict, they shall be considered to fall within the scope of description.

The above examples are merely several embodiments of the disclosure, and are specifically described in details, but cannot be interpreted as limiting the scope of the patent for the disclosure as a result. It shall be noted that for those of ordinary skill in the art, they can further make several transformations and improvements without deviating from the concept of the disclosure, and these transformations and improvements shall all fall within the scope of protection of the disclosure. Hence, the scope of protection of the patent for the disclosure shall be subject to the appended claims.

## Claims

1. A method for configuring a test project, comprising:
acquiring a program mapping relation of a target test project, wherein the program mapping relation comprises mapping relations between a plurality of target sample types and at least one target first experimental program;
acquiring program configuration information of a target second experimental program, wherein the target second experimental program uniquely corresponds to a project type of the target test project; and
configuring the target test project on the basis of the program mapping relation and the program configuration information of the target second experimental program.

2. The method as claimed in claim 1, wherein the acquiring the program mapping relation of the target test project comprises:
determining the plurality of target sample types on the basis of a sample type input operation by a user in a project configuration interface;
determining a first experimental program associated with each target sample type for each target sample type on the basis of program configuration information of first experimental programs stored in advance, and determining the target first experimental program corresponding to the target sample type from an associated first experimental program on the basis of a program selection operation by the user in the project configuration interface; and
obtaining the program mapping relation of the target test project on the basis of the plurality of target sample types determined and the target first experimental programs corresponding to the target sample types.

3. The method as claimed in claim 2,
wherein before the acquiring the program mapping relation of the target test project, the method further comprises: acquiring basic project information of the target test project on the basis of a basic information input operation by the user in the project configuration interface; and creating the target test project on the basis of the basic project information in response to a project creation operation by the user; and
the configuring the target test project on the basis of the program mapping relation and the program configuration information of the target second experimental program comprises: updating the target test project on the basis of the program mapping relation and the program configuration information of the target second experimental program.

4. The method as claimed in claim 1, wherein the acquiring the program mapping relation of the target test project comprises:
analyzing project graphic code information to obtain the program mapping relation of the target test project, wherein the project graphic code information is obtained by means of scanning by a code scanning apparatus.

5. The method as claimed in claim 4,
wherein before the configuring the target test project on the basis of the program mapping relation and the program configuration information of the target second experimental program, the method further comprises: analyzing the project graphic code information to obtain basic project information of the target test project;
the acquiring program configuration information of the target second experimental program comprises: analyzing the project graphic code information to obtain the program configuration information of the target second experimental program; and
the configuring the target test project on the basis of the program mapping relation and the program configuration information of the target second experimental program comprises: creating and configuring the target test project on the basis of the basic project information, the program mapping relation and the program configuration information of the target second experimental program.

6. The method as claimed in claim 2, further comprising:
displaying the project configuration interface, wherein the project configuration interface comprises a mode selection control, a project list area and a project editing area, wherein the mode selection control comprises an open option and a closed option;
displaying open test projects in the project list area when the mode selection control corresponds to the open option, wherein the open test projects comprise test projects created on the basis of an operation by the user in the project configuration interface;
displaying a configuration page corresponding to a selected open test project in the project editing area on the basis of a selection operation for the displayed open test projects, wherein the configuration page corresponding to the open test project comprises a configuration page of the first experimental program and a configuration page of a second experimental program;
displaying closed test projects in the project list area when the mode selection control corresponds to the closed option, wherein the closed test projects comprise test projects created by means of project graphic codes; and
displaying a configuration page corresponding to a selected closed test project in the project editing area on the basis of a selection operation for the displayed closed test projects, wherein the configuration page corresponding to the closed test project does not comprise a configuration page of the first experimental program and a configuration page of the second experimental program.

7. The method as claimed in any one of claims 1-5, wherein the method comprises at least one of the following:
obtaining and storing program configuration information of the target first experimental program on the basis of a program information input operation by the user in a program configuration interface, wherein the program configuration information comprises basic program information of the target first experimental program and experimental step information; and
analyzing program graphic code information to obtain and store program configuration information of the target first experimental program, wherein the program graphic code information is obtained by means of scanning by a code scanning apparatus; and the program configuration information comprises basic program information of the target first experimental program and experimental step information.

8. The method as claimed in claim 1, further comprising:
displaying a sample registration interface, wherein the sample registration interface displays samples to be tested and candidate test projects, and the samples to be tested correspond to target entity samples loaded in a sample analysis instrument one to one;
determining samples to be registered from the samples to be tested on the basis of a sample selection operation in the sample registration interface;
determining projects to be tested from the candidate test projects on the basis of a project selection operation in the sample registration interface; and
registering the projects to be tested for the samples to be registered in response to a registration trigger operation.

9. The method as claimed in claim 8, further comprising:
dividing the samples to be tested into experimental batches on the basis of sample types of the samples to be tested and the projects to be tested in response to an experiment starting operation, wherein the samples to be tested in the same experimental batch have the same sample type and the same project to be tested, or the samples to be tested in the same experimental batch correspond to the same first experimental program and have the same project to be tested; and
controlling the sample analysis instrument to perform corresponding experimental operations with the target entity samples corresponding to the samples to be tested in the experimental batch for each experimental batch on the basis of the sample types of the experimental batch, program mapping relations of the projects to be tested of the experimental batch, and the program configuration information of the second experimental program.

10. The method as claimed in claim 1, wherein the target first experimental program and the target second experimental program respectively refer to an operation flow of an experimental link involved in the test project.

11. A sample analysis system, comprising a sample analysis instrument and a control terminal, wherein the sample analysis instrument is communicatively connected to the control terminal;
the control terminal is configured to obtain and store a program mapping relation of a target test project and program configuration information of a target second experimental program, wherein the program mapping relation comprises mapping relations between a plurality of target sample types and at least one target first experimental program, and the target second experimental program uniquely corresponds to a project type of the target test project;
the control terminal is further configured to divide samples to be tested into experimental batches on the basis of sample types of the samples to be tested and projects to be tested, wherein the samples to be tested in the same experimental batch have the same sample type and the same project to be tested, and alternatively, the samples to be tested in the same experimental batch correspond to the same first experimental program and have the same project to be tested; and send an experimental control instruction to the sample analysis instrument for each experimental batch on the basis of the sample types of the experimental batch, program mapping relations of the projects to be tested of the experimental batch, and program configuration information of a second experimental program; and
the sample analysis instrument is configured to perform corresponding experimental operations on entity samples corresponding to the samples to be tested in each experimental batch on the basis of the experimental control instruction.

12. The sample analysis system as claimed in claim 12, wherein the control terminal is further configured to:
determine the plurality of target sample types on the basis of a sample type input operation by a user in a project configuration interface;
determine a first experimental program associated with the target sample type for each target sample type on the basis of program configuration information of first experimental programs stored in advance, and determining the target first experimental program corresponding to the target sample type from an associated first experimental program on the basis of a program selection operation by the user in the project configuration interface; and
obtain the program mapping relation of the target test project on the basis of the plurality of target sample types determined and the target first experimental programs corresponding to the target sample types

13. The sample analysis system as claimed in claim 12, wherein the control terminal is further configured to:
wherein before the acquiring the program mapping relation of the target test project, acquire basic project information of the target test project on the basis of a basic information input operation by the user in the project configuration interface; and create the target test project on the basis of the basic project information in response to a project creation operation by the user; and update the target test project on the basis of the program mapping relation and the program configuration information of the target second experimental program.

14. The sample analysis system as claimed in claim 12, wherein the control terminal is further configured to:
analyze project graphic code information to obtain the program mapping relation of the target test project, wherein the project graphic code information is obtained by means of scanning by a code scanning apparatus.

15. The sample analysis system as claimed in any one of claims 11-14, wherein the control terminal is further configured to:
display a sample registration interface, wherein the sample registration interface displays samples to be tested and candidate test projects, and the samples to be tested correspond to target entity samples loaded in a sample analysis instrument one to one;
determine samples to be registered from the samples to be tested on the basis of a sample selection operation in the sample registration interface;
determine projects to be tested from the candidate test projects on the basis of a project selection operation in the sample registration interface; and
register the projects to be tested for the samples to be registered in response to a registration trigger operation.
